# EUROPEAN PATENT SPECIFICATION

(11) **EP 3 829 604 B1**
(45) Date of publication and mention of the grant of the patent: **26.02.2025**
(21) Application number: 19840600.1
(22) Date of filing: 26.07.2019
(51) Int. Cl.: A61K 35/14, A61K 35/16, A61K 47/26, A61K 47/36, A61K 47/12, A61K 47/18, A61K 47/02, A61K 47/40, A61K 35/19, A61K 9/19, A61K 47/20, A61K 47/10, A01N 1/02, A61L 24/00

(54) **CRYOPRESERVED PLATELET COMPOSITIONS AND METHODS FOR MAKING**
KRYOKONSERVIERTE PLÄTTCHENZUSAMMENSETZUNGEN UND VERFAHREN ZU DEREN HERSTELLUNG
COMPOSITIONS DE PLAQUETTES CRYOCONSERVÉES ET LEURS PROCÉDÉS DE FABRICATION

(30) Priority: 27.07.2018 US 201862711193 P
(43) Date of publication of application: 09.06.2021
(73) Proprietor: Cellphire Inc., Rockville, MD 20850 (US)
(72) Inventor: KUHN, Benjamin J., Arlington, VA 22206 (US); AMOS, Stephen Edward, Buckeystown, Maryland 21717 (US); JORDA, Rafael, 33 700 Merignac (FR)
(74) Representative: Turner, Craig Robert
(86) International application number: PCT/US2019/043723
(87) International publication number: WO 2020/023905

(56) References cited:
- EP-A2- 1 374 890
- CN-A- 103 907 595
- US-A- 5 423 738
- US-A- 5 827 741
- US-A- 5 919 614
- US-A- 5 919 614
- US-A1- 2004 185 524
- US-A1- 2007 249 047
- US-A1- 2018 070 581
- US-B2- 8 097 403
- ANONYMOUS: "Manufacturer Specifications - CS -3000 Plus, Baxter", 19 April 2011 (2011-04-19), pages 1 - 1, XP009525951, Retrieved from the Internet <URL:https://www.google.com/search?q=inurl%3Ahttp%3A%2F%2Fwww.medwow.com%2Fmed%2F%20apheresis-machine%2Fbaxter%2Fcs-3000-plus%2F5782.model-spec&rlz=1C1SQJL_enUS861US861&oq=inurl%3Ahttp%3A%2F%2Fwww.medwow.com%2Fmed%2Fapheresis-machine%2Fbaxter%2Fcs-3000plus%2F5782.modelspec&aqs=chrome..69i57j69i58.27966j0J7&sourceid=chrome&ie=UTF-8&as_qdr=y15> [retrieved on 20190926]

## Description

### RELATED APPLICATIONS

This application claims priority to U.S. Provisional Application No. 62/711,193, filed July 27, 2018.

### TECHNICAL FIELD

The present disclosure relates to the field of blood and blood products. More specifically, it relates to cryopreserved platelet and cryopreserved platelet compositions, including those containing stabilized platelets or compositions derived from platelets.

### BACKGROUND

US 5919614 discloses a reversible inhibitor system and method that inhibits platelets from biologically activating during storage at refrigeration temperatures or freezer temperatures. US 2018/070581 A1 discloses methods and compositions relating to platelet storage media and methods for promoting prolonged storage and retention of platelet function wherein the platelet storage media includes a polyethylene glycol (PEG). EP 1374890 A2 discloses methods for enhancing platelet production in an animal through methods of administration of thrombopoietin (TPO). US 2004/185524 A1 discloses compositions and method for preserving biological samples including providing a dehydrated composition comprising dried biological sample(s) (e.g., freeze-dried platelets and cells) that are loaded with a solute to preserve biological properties during drying, freezing and rehydration. US 8097403 B2 discloses methods of making freeze-dried platelets which comprise obtaining fresh platelets from blood or a blood product, incubating the platelets in the presence of an aprotic solvent, exposing the platelets to a polysaccharide, exposing the platelets to a cryoprecipitate or a fraction thereof, and lyophilizing the platelet.

Platelets in a liquid stored apheresis form are used to treat blood-related issues, such as hemorrhages and thrombocytopenia. Typically, the platelets used in blood-related treatments have a shelf life of five days. This limits the availability of platelets for blood-related treatments. The short shelf life can also render 10-20% of available platelet collections unusable, causing about 200 million dollars to be lost annually.

Cryopreserved platelets are an alternative form of platelets. Unlike liquid-stored apheresis platelets, cryopreserved platelets can have a longer shelf life, e.g., a shelf-life of at least 6 months. Cryopreserved platelets can therefore significantly reduce waste as well as create a more viable opportunity for a medical treatment center to stock pile an inventory of cryopreserved platelets. Present cryopreservation methods employ the use of dimethyl sulfoxide (DMSO) as a cryoprotectant in a slow freezing process. However, the toxicity of DMSO prevents the usage of or requires that the DMSO be removed from thawed platelets prior to use in a patient.

There are currently no commercially available cryopreserved platelets available for human transfusion.

### SUMMARY

The present disclosure relates to the field of blood and blood products. More specifically, it relates to cryopreserved platelet and cryopreserved platelet compositions, including those containing stabilized platelets or compositions derived from platelets. As defined herein, "cryopreserved platelet composition" is a composition comprising cryopreserved platelets. Also as defined herein, "cryopreserved human platelet composition" is a composition comprising cryopreserved human platelets.

The invention is as set out in the appended claims 1 to 15.

The present invention provides a composition comprising cryopreserved platelets, wherein the cryopreserved platelets are a frozen platelet suspension; and a cryoprotectant comprising dimethyl sulfoxide (DMSO) in an amount of less than 6% v/v in the composition, wherein the cryoprotectant further comprises a combination of a polysaccharide and a disaccharide, wherein the polysaccharide is a polysucrose polymer having a concentration in the range of 3% to 10% w/v in the composition, and wherein the disaccharide is trehalose in an amount in the range of 1.5% to 5% w/v in the composition, and wherein the composition is not obtained using a lyophilization step.

Blood is a complex mixture of numerous components. In general, blood can be described as comprising four main parts: red blood cells, white blood cells, platelets, and plasma. The first three are cellular or cell-like components, whereas the fourth (plasma) is a liquid component comprising a wide and variable mixture of salts, proteins, and other factors necessary for numerous bodily functions. The components of blood can be separated from each other by various methods. In general, differential centrifugation is most commonly used currently to separate the different components of blood based on size and, in some applications, density.

Unactivated platelets, which are also commonly referred to as thrombocytes, are small, often irregularly-shaped (e.g., discoidal or ovoidal) megakaryocyte-derived components of blood that are involved in the clotting process. They aid in protecting the body from excessive blood loss due not only to trauma or injury, but to normal physiological activity as well. Platelets are considered crucial in normal hemostasis, providing the first line of defense against blood escaping from injured blood vessels. Platelets generally function by adhering to the lining of broken blood vessels, in the process becoming activated, changing to an amorphous shape, and interacting with components of the clotting system that are present in plasma or are released by the platelets themselves or other components of the blood. Purified platelets have found use in treating subjects with low platelet count (thrombocytopenia) and abnormal platelet function (thrombasthenia). Concentrated platelets are often used to control bleeding after injury or during acquired platelet function defects or deficiencies, for example those occurring during surgery and those due to the presence of platelet inhibitors. The normal human circulating platelet count is between 150,000 to 450,000 platelets per microliter (µl) of blood.

When bleeding from an injured blood vessel occurs, platelets gather at the site of injury by binding to exposed collagen on endothelial cells, and block the outflow of blood from the injured blood vessel through the process of hemostasis, which results in coagulation. Coagulation is a complex process involving platelets and multiple proteins circulating in the blood system. Further, platelets contain a number of important growth factors within their alpha granules that contribute to the process of hemostasis, coagulation, and ultimately wound healing. Studies have found that growth factors, such as platelet derived wound healing factors (PDWHF), platelet-derived growth factor (PDGF), transforming growth factor (TGF), and insulin growth factors (IGF), among others, are important in different stages of the wound healing cascade and greatly influence mitogenic and cellular differentiation activities.

As discussed above, a critical function of the blood clotting system is to stop blood loss from injured tissues, such as tissues that have been damaged by injury, wounds, surgery, or other trauma. However, sometimes the wound or trauma is so great that the blood system of the injured subject is unable to rapidly and effectively stop all of the bleeding. Furthermore, while hemostasis is provided satisfactorily in most subjects, in some subjects, hemostasis is impaired such that adequate clotting is not provided, and extensive, sometimes deadly, bleeding occurs as a result of injury, wounds, surgery, or other trauma. Thus, there are often times when a subject is in need of additional platelets or platelet-derived material to provide the clotting function that is missing or inadequate.

In addition to their use "as is" to supply blood clotting functions to subjects in need, platelets, including platelets, are studied extensively in the laboratory to characterize their properties and understand their precise role in the blood clotting cascade. Research on platelets provides information on blood clotting factors that are supplied by the platelets, factors that interact with the platelets to promote clotting and wound healing, and factors that are necessary to activate platelets or otherwise attract platelets to, and retain them at, a site of injury.

Both the therapeutic and research uses for platelets require that platelets, or compositions derived from platelets, be available in a form that is biologically active. Currently, platelets for therapeutic uses (e.g., infusion for hemostasis) are typically provided as freshly isolated products, which are less than five days old, and in some cases, preferably no more than three days old. As can be immediately recognized, maintaining an adequate supply of fresh platelets for use in subjects in need is costly and results in loss of a large amount of platelets due to expiration prior to use, particularly in rural settings and combat theaters. Furthermore, because fresh platelets are so important for use in therapy, it can be difficult and expensive to obtain fresh platelets for research purposes. The compositions and methods provided herein, however, can provide alternatives to fresh platelets for therapy and research uses in the medical and veterinary arts.

The composition includes cryopreserved platelets, and a cryoprotectant, wherein the composition comprises less than 6% v/v of dimethyl sulfoxide (DMSO).

In some embodiments, the cryopreserved platelets are cryopreserved human platelets. In some embodiments, the composition is substantially free of DMSO.

In some embodiments, the cryoprotectant is present at about 1 mM to about 1 M. In some embodiments, the cryoprotectant is present at about 10 mM 10 to about 500 mM. In some embodiments, the cryoprotectant is present at about 20 mM to about 200 mM. In some embodiments, the cryoprotectant is present at about 40 mM to about 100 mM.

The cryoprotectant comprises a combination of a polysaccharide and a disaccharide, wherein the polysaccharide is a polysucrose polymer having a concentration in the range of 3% to 10% w/v in the composition, and wherein the disaccharide is trehalose in an amount in the range of 1.5% to 5% w/v in the composition. In some embodiments not specifically claimed, the cryoprotectant comprises a component selected from the group consisting of a saccharide, a monosaccharide, a disaccharide, and combinations thereof. In some embodiments not specifically claimed, the cryoprotectant comprises a component selected from the group consisting of sucrose, maltose, trehalose, glucose, mannose, xylose, dextrose, and combinations thereof. In some embodiments, the cryoprotectant comprises a non-reducing disaccharide.

In some embodiments not specifically claimed, the cryoprotectant comprises a polymer containing one or more monosaccharide units. In some embodiments not specifically claimed, the cryoprotectant comprises a polymer derived from sucrose and epichlorohydrin. In the present invention, the cryoprotectant comprises at least a polysucrose polymer in an amount of 3% to 10% w/v of the composition. In some embodiments, the cryoprotectant is in an amount of about 5% (w/v) to about 8% (w/v). In some embodiments, the cryoprotectant comprises a high molecular weight polymer. In some embodiments, the high molecular weight polymer has an average molecular weight of at least about 70 kilodaltons (kDa).

In some embodiments not specifically claimed, the cryoprotectant further comprises serum albumin, dextran, polyvinyl pyrolidone (PVP), starch, hydroxyethyl starch (HES), and combinations thereof. In some embodiments, the cryoprotectant further comprises a sugar alcohol. In some embodiments, the sugar alcohol is selected from the group consisting of mannitol, sorbitol, xylitol, maltitol, maltitol syrup, lactitol, erythritol, isomalt, hydrogenated starch hydrolysates, serum albumin, dextran, polyvinyl pyrolidone (PVP), starch, hydroxyethyl starch (HES), and combinations thereof.

In some embodiments, the cryoprotectant comprises one or more polyols. In some embodiments, the cryoprotectant further comprises glycerol, ethylene glycol, polyethylene glycol, sorbitol, propylene glycol, pentaerythritol, a saccharide, hydroxypropyl-β-cyclodextrin, a glycerol oligomer, and combinations thereof. In some embodiments, the cryoprotectant comprises glycerol.

In some embodiments, the cryoprotectant is no more than about 10% (v/v). In some embodiments, the cryoprotectant is no more than about 5% (v/v).

**In** some embodiments, the composition also includes a solvent. **In** some embodiments, the solvent comprises water. **In** some embodiments, the composition further includes a salt. **In** some embodiments, the salt is selected from the group consisting of phosphate salts, sodium salts, potassium salts, calcium salts, magnesium salts, and combinations thereof.

**In** some embodiments, the composition also includes a buffer material. **In** some embodiments, the buffer is selected from the group consisting of phosphate buffered saline (PBS), bicarbonate/carbonic acid, such as a sodium-bicarbonate buffer, N-2-hydroxyethylpiperazine-N'-2- ethanesulfonic acid (HEPES), a tris-based buffer, a tris-buffered saline (TBS), and combinations thereof.

**In** some embodiments, the cryopreserved platelets comprises platelets derived from a human.

In some embodiments, the composition is a hemostatic composition. In some embodiments, the hemostatic composition is suitable for treating a human. In some embodiments, the hemostatic composition is suitable for triggering clotting. In some embodiments, the hemostatic composition is suitable for reducing or preventing blood loss from injured tissue.

In some embodiments, the composition is stable for at least about six months. In some embodiments, the composition is stable for at least about 12 months. In some embodiments, the composition is stable for at least about 18 months.

DMSO is present in an amount of less than 6% v/v in the composition. In some embodiments, the DMSO present in an amount of less than 5% (v/v). In some embodiments, the DMSO is present in an amount of less than 4% (v/v). In some embodiments, the DMSO is present in an amount of less than 3% (v/v). In some embodiments, the DMSO is present in an amount of less than 2% (v/v). In some embodiments, the DMSO is present in an amount of less than 1% (v/v). In some embodiments, the composition contains DMSO in an amount of less 0.001% (v/v). In some embodiments, the DMSO is in the amount of at least about 0.01% (v/v). In some embodiments, the DMSO is in the amount of at least about 0.1% (v/v). In some embodiments, the DMSO is in the amount of at least about 0.5% (v/v). In some embodiments, the DMSO is in the amount of at least about 1% (v/v). In some embodiments, the DMSO is in the amount of at least about 3% (v/v). In some embodiments, the DMSO is in the amount of about 0.01% (v/v) to about 5.5% (v/v). In some embodiments, the DMSO is in the amount of about 0.5% (v/v) to about 1% (v/v). In some embodiments, the DMSO is in the amount of about 1% (v/v) to about 3% (v/v). In some embodiments, the DMSO is in the amount of about 3% (v/v) to about 5.5% (v/v). In some embodiments, the DMSO is in the amount of about 0.5% (v/v) to about 1% (v/v).

In a second aspect, the present invention provides a method of making a cryopreserved platelet composition comprising:
obtaining platelets contained in a liquid medium;
suspending the platelets in a suspension medium comprising a cryoprotectant comprising DMSO in an amount of less than 6% v/v in the composition, wherein the cryoprotectant further comprises a combination of a polysaccharide and a disaccharide, wherein the polysaccharide is a polysucrose polymer present at 3% to 10% w/v, and wherein the disaccharide is trehalose present at 1.5% to 5% w/v to load the platelets with the cryoprotectant to form a cryoprotectant-loaded platelet suspension; and
subjecting the cryoprotectant-loaded platelet suspension to a temperature of less than - 1°C, for a time sufficient to freeze the cryoprotectant-loaded platelet suspension, thereby forming the cryopreserved platelet composition, wherein the method does not comprise a lyophilization step.

**In** some embodiments, the DMSO is in the amount of about 0.5% (v/v) to about 1% (v/v).

**In** some embodiments, the obtaining comprises isolating the platelets contained in the liquid medium. **In** some embodiments, the isolating comprises applying centrifugation filtration. **In** some embodiments, the isolating comprises applying tangential flow filtration.

**In** some embodiments, the suspension medium further comprises a salt. **In** some embodiments, the salt is present in an amount of about 0.1 mg/ml to about 10 mg/ml. **In** some embodiments, the salt is selected from the group consisting of a phosphate salt, a sodium salt, a potassium salt, a calcium salt, a magnesium salt, and combinations thereof. In some embodiments, the sodium salt is sodium chloride. **In** some embodiments, the potassium salt is potassium chloride.

In some embodiments, the suspension medium further comprises a buffering component. In some embodiments, the buffering component comprises a phosphate buffer. In some embodiments, the phosphate buffer is selected from the group consisting of a phosphate buffered saline (PBS), a bicarbonate/carbonic acid, a sodium-bicarbonate buffer, N-2-hydroxyethylpiperazine-N'-2- ethanesulfonic acid (HEPES), a tris-based buffer, a tris-buffered saline (TBS), and combinations thereof. In some embodiments, the buffering component comprises sodium bicarbonate (NaHCO3). In some embodiments, the buffering component comprises a tris-based buffer.

The cryoprotectant is as claimed.

In some embodiments, the cryoprotectant comprises glycerol. In some embodiments, the cryoprotectant is no more than about 10% (v/v).

In some embodiments, the suspension medium further comprises an organic solvent. In some embodiments, the organic solvent is present in an amount of about 0.1% (v/v) to about 2% (v/v) of the composition. In some embodiments, the organic solvent is present in an amount of about 0.1% (v/v) to about 8% (v/v) of the composition. In some embodiments, the organic solvent comprises an alcohol. In some embodiments, the alcohol comprises a short-chain alcohol. In some embodiments, the short-chain alcohol is selected from the group consisting of methanol, ethanol, propanol, 1-propanol, 2-propanol, and combinations thereof.

In some embodiments, the DMSO is present in an amount of less than 1% (v/v). In some embodiments, the DMSO is in the amount of at least about 0.1% (v/v). In some embodiments, the DMSO is in the amount of about 0.5% (v/v) to about 5% (v/v). In some embodiments, the DMSO is in the amount of about 1% (v/v) to about 3% (v/v).

In the method of the present invention, the temperature is less than -1 °C. In some embodiments, the temperature is less than about -10 °C. In some embodiments, the temperature is less than about -30 °C. In some embodiments, the temperature is less than about -70 °C. In some embodiments, the temperature is about -70°C to about -90 °C. In some embodiments, the temperature is about -10°C to about -30 °C.

In some embodiments, the platelets are human platelets.

In a third aspect, a composition is prepared by the method provided herein. In some embodiments, the composition contains DMSO in an amount of less 0.001% (v/v).

In some embodiments, the cryopreserved platelet composition comprises glycerol.

In some embodiments, the method further includes adding a pH modifier to the liquid medium containing the platelets until the pH is less than about 7. In some embodiments, the pH is less than than about 6.8. In some embodiments, the pH is about 4.0 to about 7.0. In some embodiments, the pH is about 5.0 to about 7.0. In some embodiments, the pH is about 6.6 to about 6.8.

In some embodiments, the method further includes isolating the platelets contained in the liquid medium. In some embodiments, the isolating comprises applying centrifugation, tangential flow filtration, or both, to the liquid medium. In some embodiments, the suspending comprises suspending the platelets in the suspension medium containing a concentration of 2×10⁹ platelets per milliliter (ml).

In some embodiments, the method further includes incubating the suspended platelets at a temperature of about 37°C for about 3 hours.

In some embodiments, the method further includes placing the suspended platelets in the suspension medium in a sealable container. In some embodiments, the sealable container comprises a fluorinated ethylene propylene (FEP) bag, a polyvinyl chloride (PVC) bag, or a borosilicate serum vial. In some embodiments, the suspended platelets in the suspension medium are placed in the sealable container in an amount of about 1×10⁷ platelets to about 3×10¹¹ platelets.

In some embodiments, the method further includes thawing the cryopreserved platelet composition. In some embodiments, the thawing comprising submersing the cryopreserved platelet composition in a 37°C water bath for about 8 minutes to about 10 minutes.

In some embodiments, the composition is substantially free of dimethyl sulfoxide (DMSO). In some embodiments, the composition contains DMSO in an amount of less 0.001% (v/v).

In the method of the present invention, it does not comprise a lyophilization step.

In some embodiments, the cryopreserved platelet composition comprises glycerol.

In some embodiments, the method further comprises modifying the pH of the first mixture. In some embodiments, the pH is modified to less than 7.

In some embodiments, the cryopreserved platelet composition comprises glycerol.

In some embodiments, the amount of ethanol is less than about 8% (v/v) of the composition.

In some embodiments, the cryopreserved platelet composition is substantially free of DMSO. In some embodiments, the composition is substantially free of glycerol. In some embodiments, the sum of the amounts of dimethyl sulfoxide (DMSO) and glycerol is at least about 1% (v/v). In some embodiments, the composition further comprises glycerol. In some embodiments, the glycerol is in an amount of about 0.1% (v/v) to about 10% (v/v). In some embodiments, the glycerol is in an amount of about 0.1% (v/v) to about 1% (v/v).

In some embodiments of the composition or of the method, the method includes optionally modifying the pH of the first mixture to a desired pH, for example, a pH less than 7.

In some embodiments of the composition or of the method, the method does not comprise modifying the pH of the first mixture.

The claimed method does not comprise a lyophilization step.

In some embodiments, platelets can be obtained prior to a treating step by a separation and collection of platelets, sometimes referred to as platelet apheresis (also referred to as "plateletpheresis"), in which the platelets are separated from whole blood and concentrated in a suitable container. Although most of the plasma is removed during apheresis, some plasma may still remain with the separated platelets. Apheresis platelets can be collected from a single donor and can be equivalent to about 4-6 pooled units.

In some embodiments provided herein, the platelets are human platelets and the cryopreserved platelet composition is cryopreserved human platelet composition.

In some embodiments provided herein, the platelets are not limited to platelets obtained from humans. For example, certain embodiments provided herein can include platelets obtained from other animal sources, such as canine platelets. More generally, the platelets may be obtained from an FDA-regulated platelet collection center.

Particular embodiments of the subject matter described in this document can be implemented to optionally provide one or more of the following advantages. First, some embodiments herein may provide cryopreserved platelets, or compositions containing thereof, for use in a medical treatment of a human, such as in a blood transfusion to a human. In various embodiments, the cryopreserved platelets and/or cryopreserved platelet compositions provided herein can be for use in the medical treatment of a mammalian subject, e.g., canine, or equine subjects.

Second, various embodiments provided herein provide cryopreserved platelets and/or cryopreserved platelet compositions that is free of dimethyl sulfoxide (DMSO). In some embodiments, the cryopreserved platelets and/or cryopreserved platelet compositions contain a small amount of DMSO, i.e. less than 6% v/v of the composition. For example, in some embodiments, the cryopreserved platelets and/or cryopreserved platelet composition contain an amount of DMSO that is less than 2% (v/v) of the cryopreserved platelets and/or composition. Existing cryopreserved platelet formulations are problematic because they require a large amount of DMSO. DMSO is toxic to humans (and other mammalian subjects) in large doses. The cryopreserved platelets and/or cryopreserved platelet compositions provided herein therefore provide a non-toxic, safer alternative to currently available cryopreserved platelet formulations.

Third, some embodiments provided herein include a method of cryopreserving platelets and/or platelet compositions without introducing and using dimethyl sulfoxide (DMSO) during cryopreserving. In some embodiments, provided herein is a method of cryopreserving platelets and/or platelet compositions, wherein the method does not comprise using DMSO. In some embodiments, a method of making cryopreserved platelets and/or cryopreserved platelet compositions includes using a small amount of DMSO, i.e. less than 6% v/v of the composition. For example, in some embodiments, a method of making cryopreserved platelets and/or cryopreserved platelet compositions includes using an amount of DMSO that is less than 2.% (v/v) of the cryopreserved platelets and/or composition. As discussed above, a large amount of DMSO is used for cryopreservation and can be problematic. Because DMSO is toxic to humans (and other mammalian subjects) in large doses, it would need to be removed from the platelets and platelet compositions prior to being used in a patient. Thus, a method of using lower amounts or trace amounts of DMSO to produce cryopreserved platelets and/or cryopreserved platelet compositions advantageously yields a non-toxic, safer cryopreserved platelets and platelet compositions that does not require the removal of a toxic constituent

While multiple embodiments are disclosed, still some other embodiments of the present disclosure will become apparent to those skilled in the art from the following detailed description, which shows and describes certain embodiments of the disclosure. Accordingly, the drawings and detailed description are to be regarded as exemplary in nature.

### DESCRIPTION OF DRAWINGS

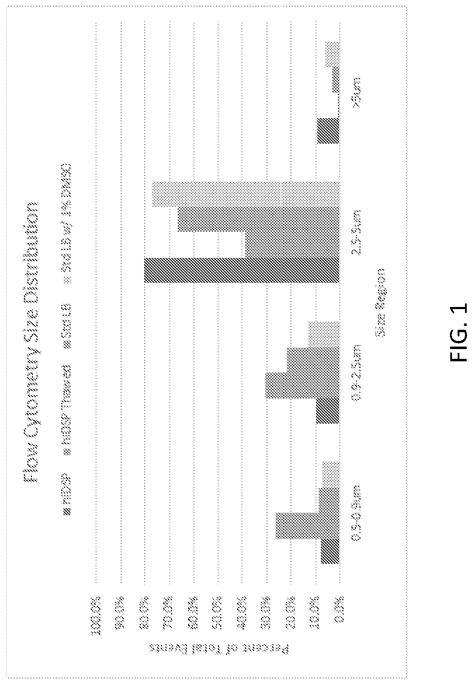

FIG. 1 is a bar graph of flow cytometry forward scattering data showing a platelet size distribution of: (i) untreated platelets ("hIDSP"); (ii) thawed untreated platelets ("hIDSP Thawed"); (iii) an exemplary cryopreserved platelet composition without DMSO ("Std LB"); and (iv) an exemplary cryopreserved platelet composition with 1 % (v/v) DMSO ("Std LB w/ 1% DMSO").

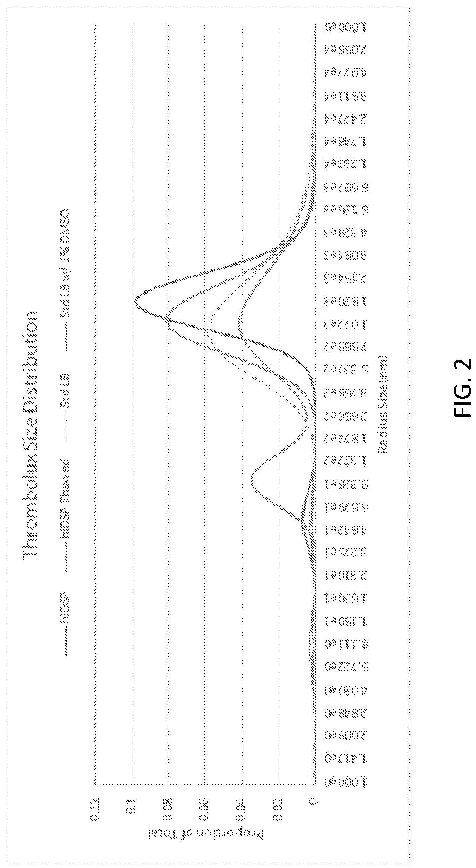

FIG. 2 is a graph plot showing size distribution data, obtained using Thrombolux Dynamic Light Scattering, of: (i) the untreated platelets (hIDSP); (ii) the thawed, untreated platelets (hIDSP Thawed); and the thawed exemplary cryopreserved platelet compositions: (iii) the exemplary platelet composition without DMSO (Std LB), and (iv) the exemplary platelet composition with 1% DMSO (Std LB w/ 1% DMSO).

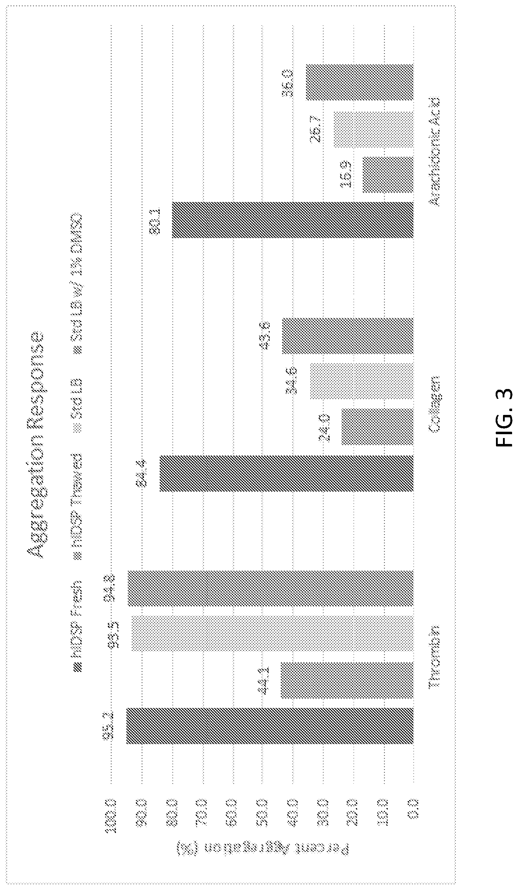

FIG. 3 is a bar graph showing the aggregation test data for (i) the untreated platelets (hIDSP); (ii) the untreated platelets (hIDSP Thawed); and the thawed exemplary cryopreserved exemplary platelet composition: (iii) the platelet composition without DMSO (Std LB), and (iv) the platelet composition with 1% DMSO (Std LB w/ 1% DMSO).

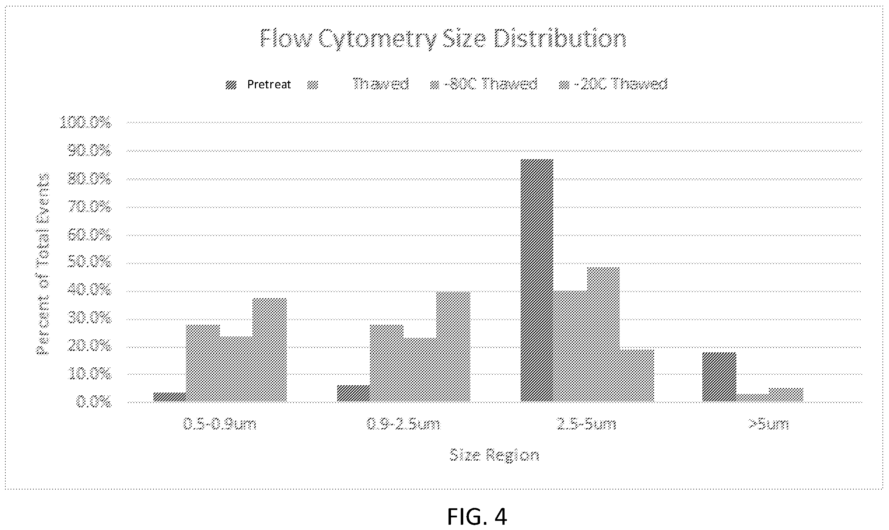

FIG. 4 is a bar graph of flow cytometry forward scattering data showing a platelet size distribution of an exemplary DMSO-free platelet composition: (i) prior to freezing ("Pretreat"); and for thawed exemplary DMSO-free platelet compositions that had been frozen at (ii) -1°C/minute ("Thawed"), (iii) -80°C ("-80C Thawed"), or (iii) - 20°C ("-20C Thawed").

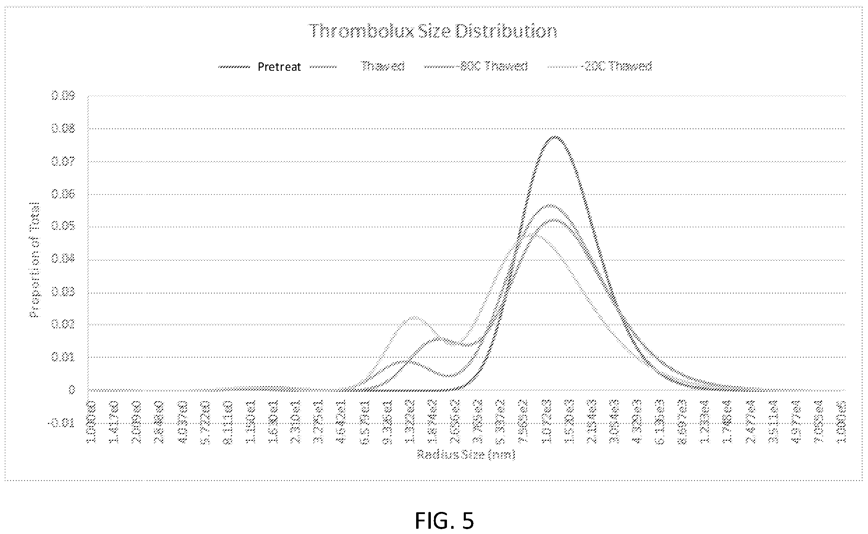

FIG. 5 is a graph plot of Thrombolux dynamic light scattering data showing the size distributions of the exemplary DMSO-free platelet composition prior to freezing ("Pretreat") and for the thawed exemplary DMSO-free platelet compositions frozen at -1°C/minute ("Thawed"), -80°C ("-80C Thawed"), or -20°C ("-20C Thawed").

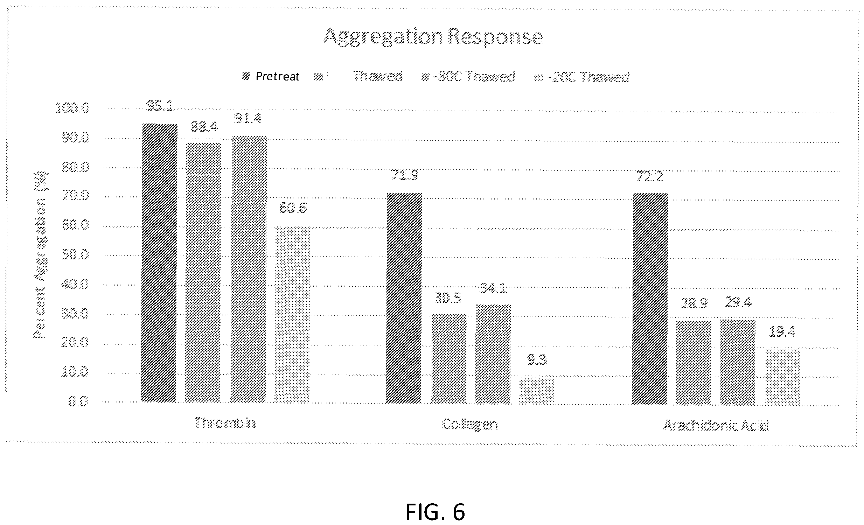

FIG. 6 is a bar graph showing the aggregation test results for the exemplary DMSO-free platelet composition prior to freezing ("Pretreat") and for the thawed exemplary DMSO-free platelet compositions frozen at -1°C/minute ("Thawed"), -80°C ("-80C Thawed"), or -20°C ("-20C Thawed").

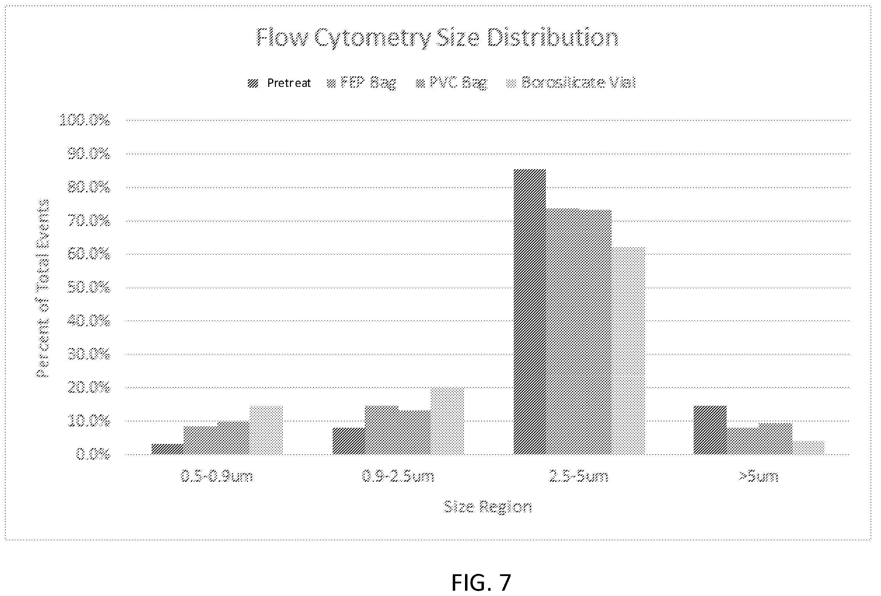

FIG. 7 is a bar graph of flow cytometry forward scattering data showing a platelet size distribution of an exemplary DMSO-free platelet composition at (i) prior to freezing ("Pretreat"); and for exemplary thawed DMSO-free platelet compositions subjected to a temperature of -80°C while contained in (ii) a FEP bag, (iii) PVC bag, or (iii) a borosilicate vial.

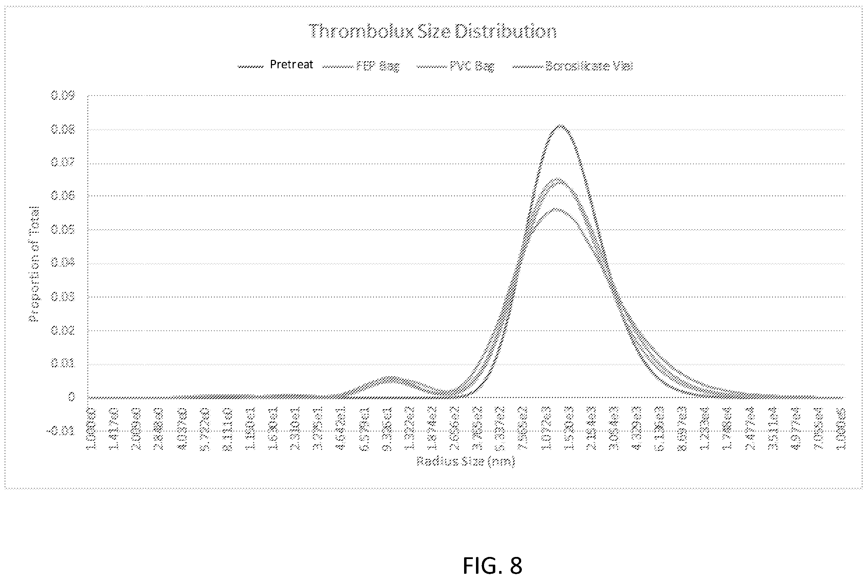

FIG. 8 provides a graph plot of Thrombolux dynamic light scattering data showing the size distributions of the exemplary DMSO-free platelet composition prior to freezing ("Pretreat") and for the exemplary thawed DMSO-free platelet compositions subjected to a temperature of -80°C while contained in a FEP bag, PVC bag, or borosilicate vial.

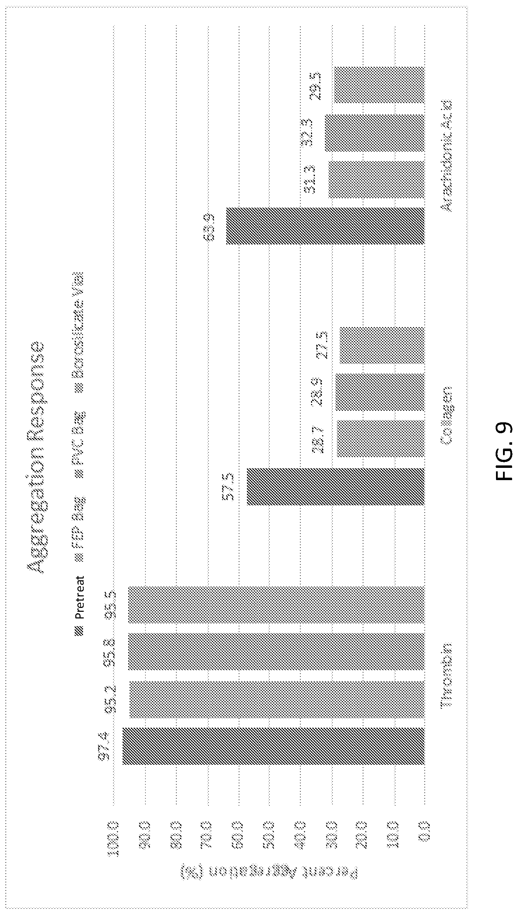

FIG. 9 is a bar graph showing aggregation test results for exemplary DMSO-free platelet composition compositions prior to freezing ("Pretreat") and for the exemplary thawed DMSO-free platelet compositions subjected to a temperature of -80°C while contained in a FEP bag, PVC bag, or borosilicate vial.

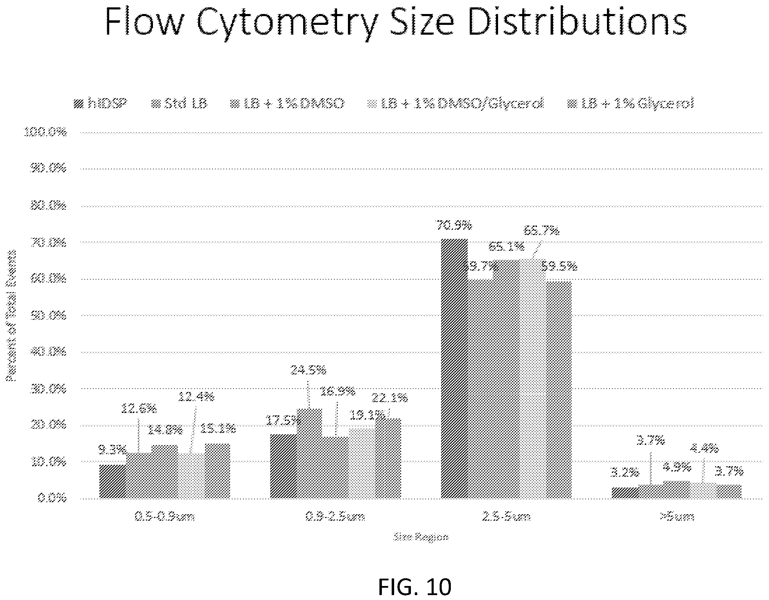

FIG. 10 is a bar graph of flow cytometry forward scattering data showing a platelet size distribution of: (i) untreated platelets ("hIDSP"); and the cryopreserved platelet compositions, including: (ii) the DMSO-free platelet composition ("Std LB"); (iii) the platelet composition with 1% DMSO ("LB + 1% DMSO"); (iv) the platelet composition with 1% DMSO and 1% glycerol ("LB + 1% DMSO/Glycerol"); and (v) the platelet composition with 1% glycerol ("LB + 1% glycerol).

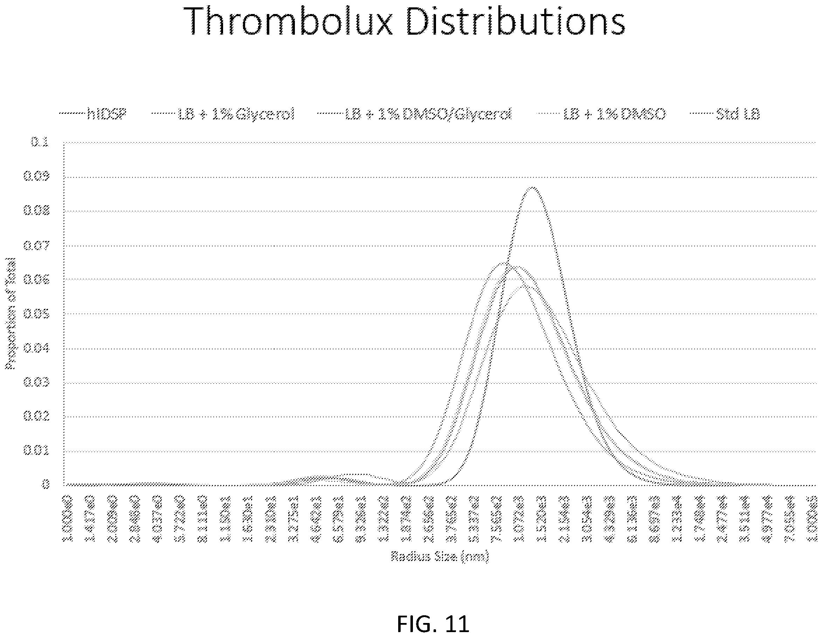

FIG. 11 is a graph plot showing size distribution data, obtained using Thrombolux Dynamic Light Scattering, of: (i) the untreated platelets (hIDSP) and the cryopreserved platelet compositions, including: (ii) the DMSO-free platelet composition ("Std LB"); (iii) the platelet composition with 1% DMSO ("LB + 1% DMSO"); (iv) the platelet composition with 1% DMSO and 1% glycerol ("LB + 1% DMSO/Glycerol"); and (v) the platelet composition with 1% glycerol ("LB + 1% glycerol).

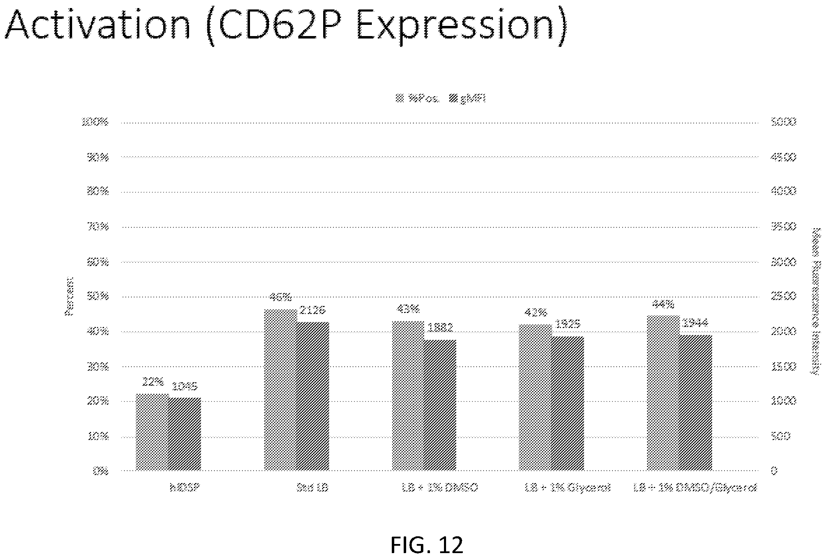

FIG. 12 is a graph plot showing platelet activation test results specific to CD62P expression, of: (i) the untreated platelets (hIDSP) and the cryopreserved platelet compositions, including: (ii) the DMSO-free platelet composition ("Std LB"); (iii) the platelet composition with 1% DMSO ("LB + 1% DMSO"); (iv) the platelet composition with 1% DMSO and 1% glycerol ("LB + 1% DMSO/Glycerol"); and (v) the platelet composition with 1% glycerol ("LB + 1% glycerol).

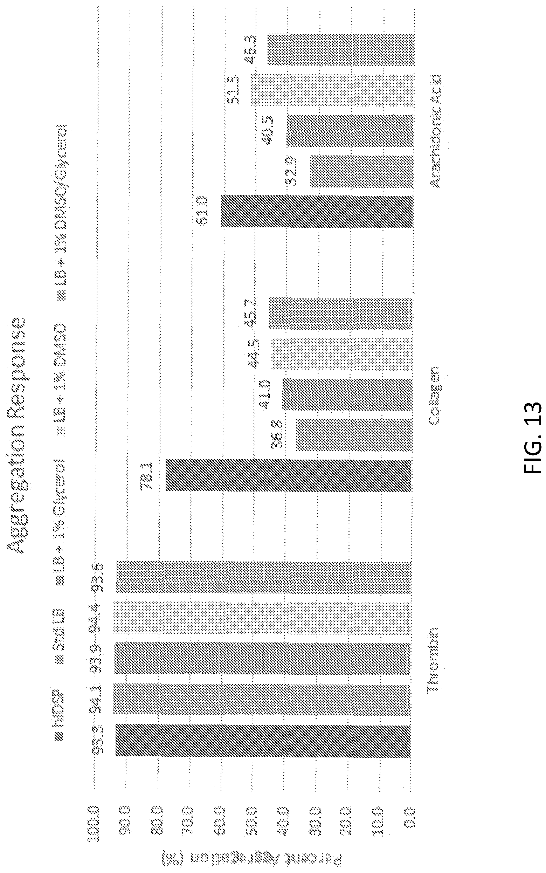

FIG. 13 is a bar graph showing the aggregation test data for (i) the untreated platelets (hIDSP) and the cryopreserved platelet compositions, including: (ii) the DMSO-free platelet composition ("Std LB"); (iii) the platelet composition with 1% DMSO ("LB + 1% DMSO"); (iv) the platelet composition with 1% DMSO and 1% glycerol ("LB + 1% DMSO/Glycerol"); and (v) the platelet composition with 1% glycerol ("LB + 1% glycerol).

### DETAILED DESCRIPTION

The present disclosure relates to the field of blood and blood products. More specifically, it relates to cryopreserved platelets and cryopreserved platelet compositions, including those containing stabilized platelets or compositions derived from platelets. In some embodiments, the present disclosure provides cryopreserved platelets and cryopreserved platelet compositions containing a small amount of DMSO (e.g., less than 2% (v/v) of DMSO), or the present disclosure provides cryopreserved platelets and cryopreserved platelet compositions containing no DMSO.

The present invention provides a composition comprising cryopreserved platelets, wherein the cryopreserved platelets are a frozen platelet suspension; and a cryoprotectant comprising dimethyl sulfoxide (DMSO) in an amount of less than 6% v/v in the composition, wherein the cryoprotectant further comprises a combination of a polysaccharide and a disaccharide, wherein the polysaccharide is a polysucrose polymer having a concentration in the range of 3% to 10% w/v in the composition, and wherein the disaccharide is trehalose in an amount in the range of 1.5% to 5% w/v in the composition, and wherein the composition is not obtained using a lyophilization step.

The present invention also provides provides a method of making a cryopreserved platelet composition comprising: obtaining platelets contained in a liquid medium; suspending the platelets in a suspension medium comprising a cryoprotectant comprising DMSO in an amount of less than 6% v/v in the composition, wherein the cryoprotectant further comprises a combination of a polysaccharide and a disaccharide, wherein the polysaccharide is a polysucrose polymer present at 3% to 10% w/v, and wherein the disaccharide is trehalose present at 1.5% to 5% w/v to load the platelets with the cryoprotectant to form a cryoprotectant-loaded platelet suspension; and subjecting the cryoprotectant-loaded platelet suspension to a temperature of less than -1°C, for a time sufficient to freeze the cryoprotectant-loaded platelet suspension, thereby forming the cryopreserved platelet composition, wherein the method does not comprise a lyophilization step.

In some embodiments provided herein, the platelets are human platelets and the cryopreserved platelet composition is cryopreserved human platelet composition.

In the composition or the method of the present invention, the method does not comprise a lyophilization step.

Each platelet has an intracellular domain and an extracellular domain. As used herein, "intracellular" refers to the domain that is inside a cell membrane, e.g., within a platelet membrane, and "extracellular refers to the domain that is outside of the cell membrane, e.g., outside of the platelet membrane. In some embodiments, the intracellular amount of trehalose in cryopreserved platelets correlates to an intracellular concentration of trehalose that is about 0.5 to about 20 mM, or about 0.5 to about 10 mM in the cryopreserved platelets. In some embodiments, the intracellular concentration of trehalose in cryopreserved platelets is more than about 0.5 mM (e.g., more than about 1.0 mM, about 1.5 mM, about 2.0 mM, about 2.5 mM, about 3.0 mM, about 4.0 mM, about 4.5 mM, about 5.0 mM, about 6.0 mM, about 7.0 mM, about 8 mM, about 9 mM, about 10 mM, about 11 mM, about 12 mM, about 13 mM, about 14 mM, or more than about 15 mM). In some embodiments, the total concentration of trehalose in the cryopreserved platelet is more than about 0.5 mM (e.g., more than about 1.0 mM, about 1.5 mM, about 2.0 mM, about 2.5 mM, about 3.0 mM, about 4.0 mM, about 4.5 mM, about 5.0 mM, about 6.0 mM, about 7.0 mM, about 8 mM, about 9 mM, about 10 mM, about 11 mM, about 12 mM, about 13 mM, about 14 mM, or more than about 15 mM). In some more particular embodiments, the intracellular amount and/or concentration of trehalose in the cryopreserved platelets as disclosed herein is obtained by mixing platelets with trehalose in an amount of about 80 mM.

In some embodiments, the predetermined time of cooling a mixture comprising platelets and a cryoprotectant is about 1 hour to about 6 hours (e.g., from about 1 hour to about 5 hours, from about 1 hour to about 4 hours, from about 1 hour to about 3 hours, from about 1 hour to about 2 hours, from about 2 hours to about 6 hours, from about 2 hours to about 5 hours, from about 2 hours to about 4 hours, from about 2 hours to about 3 hours, from about 3 hours to about 6 hours, from about 3 hours to about 5 hours, from about 3 hours to about 4 hours, from about 4 hours to about 6 hours, from about 4 hours to about 5 hours, or from about 5 hours to about 6 hours). In some embodiments, the predetermined time is less than about 6 hours (e.g., less than about 5 hours, less than about 4 hours, less than about 3 hours, less than about 2 hours, or less than about 1 hour). In some embodiments, is more than about 1 hour (e.g., more than 2 hours, more than about 3 hours, more than about 4 hours, more than about 5 hours, or more than about 6 hours). In some more particular embodiments, the intracellular concentration of trehalose in the cryopreserved platelets is as disclosed herein and the total concentration of trehalose in the cryopreserved platelets is about 80 mM. In some embodiments, the intracellular amount of trehalose in the cryopreserved platelets is about 0.5 to about 20 mM, or about 0.5 to about 10 mM, following a predetermined time of cooling a mixture comprising platelets and trehalose of about 2 hours to about 6 hours, or about 3 hours to about 5 hours, or about 4 hours.

In some embodiments, the predetermined time to form the cryopreserved platelet composition is about 1 hour to about 48 hours. In some embodiments, the predetermined time to form the cryopreserved platelet composition is about 3 hours to about 36 hours, about 6 hours to about 24 hours, about 12 hours to about 24 hours, about 24 hours to about 36 hours, or about 24 hours to about 48 hours. In some embodiments, the predetermined time to form the cryopreserved platelet composition is more than about 1 hour (e.g., more than about 3 hours, about 6 hours, about 12 hours, about 24 hours, about 36 hours or about 48 hours). In some embodiments, the predetermined time to form the cryopreserved platelet composition is less than about 48 hours (e.g., less than about 36 hours, about 24 hours, about 12 hours, about 6 hours, about 3 hours, or less than one hour).

The cryopreserved platelet composition comprises 3% to 10% w/v of polysucrose and 1.5% to 5% w/v of trehalose. In some embodiments, the cryopreserved platelet composition comprises polysucrose and trehalose in a ratio of about 1:1 to about 5:1 (e.g., from about 1:1 to about 4:1, from about 1:1 to about 3:1, from about 1:1 to about 2:1, from about 2:1 to about 5:1, from about 3:1 to about 5:1, from about 4:1 to about 5:1, from about 3:1 to about 5:1, or from about 4:1 to about 5:1). In some embodiments, the cryopreserved platelet composition comprises polysucrose and trehalose in a ratio in a range from about 1:1 to about 2:1, about 3:1, about 4:1, about 5:1, about 6:1; from about 2:1 to about 3:1, about 4:1, about 5:1, about 6:1; from about 3:1 to about 4:1, about 5:1, about 6:1; from about 4:1 to about 5:1, about 6:1; from about 5:1 to about 6:1. In some embodiments, the cryopreserved platelet composition comprises polysucrose and trehalose in a ratio in a range from about 1:1 to about 1:2, about 1:3, about 1:4, about 1:5, about 16; from about 1:2 to about 1:3, about 1:4, about 1:5, about 16; from about 1:3 to about 1:4, about 1:5, about 16; from about 1:4 to about 1:5, about 16; from about 1:5 to about 1:6.

In some embodiments, the cryopreserved platelet composition comprises about 6% (w/v) of polysucrose and about 3% (w/v) of trehalose.

As used herein, (w/v) refers to "weight over volume" and (v/v) refers to "volume over volume".

Herein is also described improved compositions for use in treating wounds by hemostasis and treating coagulopathy, and methods of making such compositions. Herein described is compositions for use in treating wounds to stop blood loss that are rapid, effective, and suitable for use in multiple settings.

Various embodiments in the present disclosure include cryopreserved platelet compositions (which may also be referred to as "platelet composition"), derived from human platelets, that can be applied in the medical art for medical treating. The cryopreserved platelet compositions are prepared according to claim 7. In embodiments, the method for preparation of the platelet compositions further includes thawing the cryopreserved platelet compositions. The cryopreserved platelet compositions can be used for in numerous purposes, including, but not limited to, use as hemostatic agents to form clots at sites of injury involving bleeding, use for in treating coagulopathy, and use for in promoting tissue regeneration and healing. The present disclosure also describes compositions for use in preventing or treating expected or active excessive bleeding associated with anticoagulant therapy or other therapies or environmental effects that result in inhibition of the clotting cascade. The present disclosure also addresses needs in the art by describing compositions for use in diagnostics for detection of blood clotting disorders. Accordingly, the present disclosure describes methods for making diagnostic compositions for use in diagnosing bleeding disorders.

The present disclosure further addresses needs in the medical art by providing methods for preparing human cryopreserved platelet compositions for storage and/or for use. For example, preparing the human cryopreserved platelet compositions for storage can include subjecting the compositions to a low temperature i.e. less than -1°C and freezing the compositions. Preparing the cryopreserved platelet compositions for use can include subjecting the compositions to a high temperature (e.g., greater than 1°C), partially thawing, and/or fully thawing the compositions. Methods of this disclosure provide cryopreserved platelets that are stable for extended periods of time at a wide range of temperatures. The methods, and the compositions, also provide platelets that, upon thawing (and optionally reconstitution), function well in the process of blood clotting, and thus can be used successfully in therapeutic applications, such as for wound healing and treatment of bleeding diseases and disorders in humans. Kits are provided to contain the cryopreserved platelet compositions.

In some embodiments, the present disclosure may address needs in the veterinary art by providing cryopreserved platelet compositions derived from animal (e.g., canine) platelets.

Cryopreserved platelet compositions provided herein include platelets, and/or platelet-derived materials. In some embodiments, the cryopreserved platelet composition comprises human platelets. In some embodiments, the cryopreserved platelet compositions provided herein are derived from human platelets. In some embodiments, the cryopreserved platelet compositions provided herein are derived from the platelets of other animals, such as canine platelets.

The cryopreserved platelet composition can be in a solid form (e.g., after freezing a cryopreserved platelet composition), a liquid form (e.g., after thawing a frozen cryopreserved platelet composition), or combinations thereof (e.g., partially solid (frozen), partially liquid). In some embodiments, the cryopreserved platelet composition contains less than about 10 percent (<10 wt.%), less than five percent (< 5 wt.%), or less than two percent (< 2 wt.%) residual moisture.

The cryopreserved platelet composition, when in liquid form, can include the thawed platelets. The liquid portion of the composition can also include water, an aqueous liquid, blood or a blood component or fraction (such as plasma), saline, buffered saline (e.g., phosphate buffered saline), or the like. In some embodiments, the cryopreserved platelet composition is sterile and has less than two Endotoxin Units (EU) per milliliter (ml) when in liquid form. The liquid form of the cryopreserved platelet composition provided herein can include at least about 50%, or at least about 70% of the particles in the range of about 0.5 µm to about 0.9 µm.

In various embodiments, the cryopreserved platelet composition (e.g., a frozen composition, a liquid composition, or combination thereof) contains a small amount of DMSO less than 6% v/v, or is substantially free of DMSO. For example, in some embodiments, the cryopreserved platelet composition contains an amount of less about 0.001% (v/v) of DMSO in the composition. In some embodiments, the cryopreserved platelet composition includes cryopreserved platelets and a cryoprotectant, wherein the cryopreserved platelet composition is substantially free of dimethyl sulfoxide (DMSO). The cryopreserved platelet composition includes cryopreserved platelets, and a cryoprotectant comprising dimethyl sulfoxide (DMSO) in an amount of less 6% (v/v). In some embodiments, the cryopreserved platelet composition includes DMSO present in an amount of less than about 5%, about 4%, about 3%, about 2%, or about 1% (v/v). In some embodiments, the cryopreserved platelet composition includes DMSO present in an amount of at least about 0.001%, about 0.01%, about 0.1%, or about 0.5% (v/v) and less than 6% v/v. In some embodiments, the cryopreserved platelet composition includes DMSO is in the amount of about 0.01% (v/v) to about 5% (v/v), about 0.01% (v/v) to about 4% (v/v), about 0.01% (v/v) to about 3% (v/v), about 0.01% (v/v) to about 2% (v/v), or about 0.01% (v/v) to about 1% (v/v), about 0.5% (v/v) to about 5% (v/v), about 0.5% (v/v) to about 4% (v/v), about 0.5% (v/v) to about 3% (v/v), about 0.5% (v/v) to about 2% (v/v), or about 0.5% (v/v) to about 1% (v/v).

It has been surprisingly found that the cryopreserved platelet compositions containing a low amount of DMSO (such as from about 0.5% v/v to about 5% v/v) exhibit desirable platelet function (e.g., size distribution, yield, and aggregation characteristics) as compared to the untreated platelets. As used in this document, "untreated platelets" refers to platelets contained in their original collection liquid medium, for example, an apheresis liquid medium that may contain plasma and/or water. In some embodiments, cryopreserved platelet compositions that are substantially free of DMSO exhibit acceptable platelet function (e.g., size distribution, yield, and aggregation characteristics) as compared to the untreated platelets. Similarly, it has been surprisingly found that cryopreserved platelet compositions containing a low amount of DMSO exhibit desirable platelet function (e.g., size distribution, yield, and aggregation characteristics) as compared to the untreated platelets. In some embodiments, cryopreserved platelet compositions that are substantially free of DMSO also exhibit desirable platelet function (e.g., size distribution, yield, and aggregation characteristics) as compared to the untreated platelets. The cryopreserved platelet compositions provided herein can be highly advantageous because having a reduced amount (or eliminating the presence) of DMSO, which can be toxic in high amounts, yields compositions that can be safely administered to a patient. Furthermore, the cryopreserved platelet compositions provided herein can be highly advantageous because no additional processing, e.g., removing DMSO prior to use, is necessary for the compositions to be safely administered to a patient if they contain a sufficiently low amount of DMSO or no DMSO.

In some embodiments, the cryopreserved platelet composition provided herein includes platelets and/or platelet-derived particles having a particle size (e.g., diameter, max dimension) of at least about 0.2 µm (e.g., at least about 0.3 µm, at least about 0.4 µm, at least about 0.5 µm, at least about 0.6 µm, at least about 0.7 µm, at least about 0.8 µm, at least about 0.9 µm, at least about 1.0 µm, at least about 1.0 µm, at least about 1.5 µm, at least about 2.0 µm, at least about 2.5 µm, or at least about 5.0 µm). In some embodiments, the cryopreserved platelet composition provided herein includes platelets having a particle size of less than about 5.0 µm (e.g., less than about 2.5 µm, less than about 2.0 µm, less than about 1.5 µm, less than about 1.0 µm, less than about 0.9 µm, less than about 0.8 µm, less than about 0.7 µm, less than about 0.6 µm, less than about 0.5 µm, less than about 0.4 µm, or less than about 0.3 µm). In some embodiments, the cryopreserved platelet composition provided herein includes platelets having a particle size of from about 0.3 µm to about 5.0 µm (e.g., from about 0.4 µm to about 4.0 µm, from about 0.5 µm to about 2.5 µm, from about 0.6 µm to about 2.0 µm, from about 0.7 µm to about 1.0 µm, from about 0.5 µm to about 0.9 µm, or from about 0.6 µm to about 0.8 µm).

In some embodiments, a cryopreserved platelet composition has at least 50% (e.g., at least about 55%, at least about 60%, at least about 65%, at least about 70%, at least about 75%, at least about 80%, at least about 85%, at least about 90%, at least about 95%, or at least about 99%) of platelets and/or platelet-derived particles in the range of about 0.3 µm to about 5.0 µm (e.g., from about 0.4 µm to about 4.0 µm, from about 0.5 µm to about 2.5 µm, from about 0.6 µm to about 2.0 µm, from about 0.7 µm to about 1.0 µm, from about 0.5 µm to about 0.9 µm, or from about 0.6 µm to about 0.8 µm). In some embodiments, the cryopreserved platelet composition has at most 99% (e.g., at most about 95%, at most about 80%, at most about 75%, at most about 70%, at most about 65%, at most about 60%, at most about 55%, or at most about 50%) of platelets and/or platelet-derived particles in the range of about 0.3 µm to about 5.0 µm (e.g., from about 0.4 µm to about 4.0 µm, from about 0.5 µm to about 2.5 µm, from about 0.6 µm to about 2.0 µm, from about 0.7 µm to about 1.0 µm, from about 0.5 µm to about 0.9 µm, or from about 0.6 µm to about 0.8 µm). In some embodiments, the cryopreserved platelet composition has about 50% to about 99% (e.g., about 55% to about 95%, about 60% to about 90%, about 65% to about 85, about 70% to about 80%) of platelets and/or platelet-derived particles in the range of about 0.3 µm to about 5.0 µm (e.g., from about 0.4 µm to about 4.0 µm, from about 0.5 µm to about 2.5 µm, from about 0.6 µm to about 2.0 µm, from about 0.7 µm to about 1.0 µm, from about 0.5 µm to about 0.9 µm, or from about 0.6 µm to about 0.8 µm).

In therapeutic use, the cryopreserved platelet composition retains a sufficient level of components necessary for the blood clotting function of platelets when introduced into subjects in need of platelet functions. The cryopreserved platelet composition can comprise other blood components, and in particular can comprise blood clotting factors, such as Factor VII and Factor VIII, in their normal or activated states. These other components may be present as a result of concentrating of the platelets or they may be added as separately purified components to the platelets prior to or after subjecting the platelets to a low temperature or freezing the platelets. These other blood components may be present singly (i.e., only one is present in the composition), or multiple other blood components may be included in the composition together with the platelets and/or platelet-derived particles. Typically, the other blood components are included in amounts or concentrations that, when administered to a subject, provide a detectable change in at least one physiological process of the treated subject, or provide a known benefit.

In various embodiments, the cryopreserved platelet composition provided herein has a pH of less than about 5.0, about 5.5, about 6.0, about 6.5, about 7.0, about 7.5, or about 8.0. In some embodiments, the cryopreserved platelet composition provided herein has a pH range of about 3.0 to about 9.0, about 4.0 to about 8.0, about 5.0 to about 7.0, about 6.0 to about 7.5, or about 6.5 to about 7.0, during the method of preparation. In some embodiments, a cryopreserved platelet composition has a pH that is less than about 10.0 (e.g., less than about 9.5, about 9.0, about 8.5, about 8.0, about 7.5, about 7.0, about 6.5, about 6.0, about 5.5, about 5.0, about 4.5, about 4.0, about 3.5, about 3.0, or less).

The cryopreserved platelet composition is made by the processes described herein. The method includes a first step of obtaining a composition containing platelets in a liquid medium, such as a liquid medium (e.g., water, plasma, or the like). Platelets can be obtained from pooled platelet concentrates, by separation of platelet concentrates from whole blood, by plateletphresis, or combinations thereof.

The method can include acidifying the platelets and the liquid medium to a desired pH level. For example, in some embodiments, a pH modifier can be added to the platelets and the liquid medium to lower the pH of the composition to no more than about 5.0, about 5.5, about 6.0, about 6.5, about 7.0, about 7.5, or about 8.0. In some embodiments, the pH modifier is added in an amount such that the composition has a pH range of about 6.0 to about 9.0, about 6.2 to about 8.0, about 6.6 to about 6.8, or about 6.4 to about 7.4. In some embodiments, the pH modifier includes an acid citrate dextrose (ACD) composition. In some embodiments, the ACD composition can contain sodium citrate, citric acid, dextrose, or combinations thereof. In some embodiments, the sodium citrate is present at about 0.01 M to about 0.8 M (e.g., from about 0.03 M to about 0.5 M, or from about 0.05 M to about 0.1 M) in the ACD composition. In some embodiments, the ACD composition can include sodium citrate in an amount of about 3 mg to about 250 mg (e.g., about 5 mg to about 200 mg, about 10 mg to about 100 mg, or about 15 mg to about 50 mg) per milliliter (mL) in the ACD composition. In some embodiments, the sodium citrate is present at about 0.01 M to about 0.7 M (e.g., from about 0.03 M to about 3.0 M, or from about 0.05 M to about 1.0 M) in the composition. In some embodiments, the ACD composition can include sodium citrate in an amount of about 3 mg to about 250 mg) per mL of the ACD composition. In some embodiments, the dextrose is present at about 0.01 M to about 1.5 M (e.g., from about 0.03 M to about 1.0 M, from about 0.05 M to about 0.5 M, or from about 0.8 M to about 0.3 M) in the composition. In some embodiments, the ACD composition can include sodium citrate in an amount of about 2 mg to about 200 mg (e.g., about 5 mg to about 100 mg, about 10 mg to about 50 mg, or about 15 mg to about 25 mg) per mL of the ACD composition.

The method can include isolating the platelets, for example, by purifying the platelets to a desired extent to form platelet rich plasma (PRP). The step of purifying the platelets can use any method known in the art as useful for obtaining purified platelets, including centrifugation (such as differential centrifugation) and filtration (e.g., tangential flow filtration). In some embodiments, plateletpheresis can be used to provide PRP.

The method for making the cryopreserved platelet composition provided herein includes suspending the purified platelets in a suspension medium. The suspension medium includes a cryoprotectant for a sufficient amount of time and at a suitable temperature to allow for entry of the cryoprotectant into the platelets (also referred to herein as "loading" the platelets), as disclosed in claim 7. Accordingly, the first mixture referred to herein comprises platelets and the suspension medium comprising the cryoprotectant. Without applicants being bound by any theory or mechanism, the cryoprotectant is thought to stabilize proteins and other biological substances in the interior of the platelets. The identity of the cryoprotectant is not limited as long as it can enter the platelets and provide a cryoprotectant property. In some embodiments, the suspension medium includes a cryoprotectant that comprises DMSO but in amounts less than 6% v/v. In some embodiments, the suspension medium is substantially free of DMSO.

The cryoprotectant may include further saccharides, such as monosaccharides and disaccharides, including sucrose, maltose, glucose, mannose, dextrose, xylose, and combinations thereof. The saccharide for use in the method of preparing a cryopreserved platelet composition provided herein comprises trehalose.

In some embodiments, a suitable cryoprotectant further includes one or more sugar alcohols. Non-limiting examples of sugar alcohols can include mannitol, sorbitol, xylitol, maltitol, maltitol syrup, lactitol, erythritol, and combinations thereof.

In some embodiments, the further saccharide can be present at about 1 mM to about 1 M. In embodiments, the further saccharide is present at about 10 mM to about 500 mM. In some embodiments, the further saccharide is present at about 20 mM to about 200 mM. In embodiments, the further saccharide is present at about 40 mM to about 100 mM. In some embodiments, one or more further saccharides are present in about 0.04 mg/ml to about 4 mg/ml, about 0.04 mg/ml to about 0.4 mg/ml, or about 0.4 mg/ml to about 4 mg/ml in the composition. In some embodiments, one or more further saccharides are present in about 3 mg/ml to about 300 mg/ml, about 3 mg/ml to about 30 mg/ml, or about 30 mg/ml to about 300 mg/ml in the composition.

In various embodiments, the further saccharide is present in different specific concentrations within the ranges recited above, and one of skill in the art can immediately understand the various concentrations without the need to specifically recite each herein. Where more than one further saccharide is present in the composition, each saccharide can be present in an amount according to the ranges and particular concentrations recited above.

In some embodiments, the cryoprotectant includes one or more polyols. In some embodiments, suitable cryoprotectants can include glycerol (glycerin), ethylene glycol, polyethylene glycol, sorbitol, propylene glycol, pentaerythritol, a saccharide, hydroxypropyl-β-cyclodextrin, a glycerol oligomer, or combinations thereof. In some embodiments, the cryoprotectant is no more than about 10% (v/v) (e.g., no more than about 9% (v/v), 8% (v/v), 7% (v/v), 6% (v/v), 5% (v/v). In some embodiments, the cryoprotectant is in an amount of at least about 5% (v/v), 6% (v/v), 7% (v/v), 8% (v/v), 9% (v/v), or 10% (v/v). In some embodiments, the cryoprotectant is in an amount up to about 10% (v/v). In some embodiments, the cryoprotectant is in an amount of up to about 7% (v/v). In some embodiments, the cryoprotectant is in an amount of up to about 5% (v/v). In some embodiments, the glycerol is in an amount of about 0.1% (v/v) to about 1% (v/v). Glycerol provides beneficial properties as an antifreeze agent. It can also serve as a humectant, in some embodiments.

In some embodiments, the method includes suspending the purified platelets in an aqueous suspension medium that is buffered with one or more buffering components. The buffering component can be any buffer that is non-toxic to the platelets and provides adequate buffering capacity to the solution at the temperatures at which the solution will be exposed during the method provided herein. Thus, the buffer may comprise any of the known biologically compatible buffers available commercially, such as phosphate buffers, such as phosphate buffered saline (PBS), bicarbonate/carbonic acid, such as sodium-bicarbonate buffer, N-2-hydroxyethylpiperazine-N'-2- ethanesulfonic acid (HEPES), and tris-based buffers, such as tris-buffered saline (TBS). Likewise, it may comprise one or more of the following buffers: propane- 1,2,3-tricarboxylic (tricarballylic); benzenepentacarboxylic; maleic; 2,2- dimethylsuccinic; EDTA; 3,3-dimethylglutaric; bis(2-hydroxyethyl)imino- tris(hydroxymethyl)-methane (BIS-TRIS); benzenehexacarboxylic (mellitic); N-(2- acetamido)imino-diacetic acid (ADA); butane-1,2,3,4-tetracarboxylic; pyrophosphoric; 1,1-cyclopentanediacetic (3,3 tetramethylene-glutaric acid); piperazine-1,4-bis-(2-ethanesulfonic acid) (PIPES); N-(2-acetamido )-2- amnoethanesulfonic acid (ACES); 1,1-cyclohexanediacetic; 3,6-endomethylene- 1,2,3,6-tetrahydrophthalic acid (EMTA; ENDCA); imidazole;; 2-(aminoethyl)trimethylammonium chloride (CHOLAMINE); N,N-bis(2-hydroxyethyl)-2-aminoethanesulfonic acid (BES); 2-methylpropane-1,2,3-triscarboxylic (beta-methyltricarballylic ); 2-(N-morpholino)propane-sulfonic acid (MOPS); phosphoric; and N-tris(hydroxymethyl)methyl-2-amminoethane sulfonic acid (TES).

**In** various embodiments, one or more buffering components can be present in the composition in any suitable amount. **In** some embodiments, the buffering component can be present in an amount of 1 mM to 1 M. **In** some embodiments, one or more buffering components are present in about 0.2 to about 20 mg/ml, or about 0.2 to about 2 mg/ml, or about 2 mg/ml to about 20 mg/ml in the composition. **In** some embodiments, one or more salts are present in about 0.08 to about 8 mg/ml, such as about 0.08 to about 0.8 mg/ml, or about 0.8 mg/ml to about 8 mg/ml in the composition.

The method of preparing the cryopreserved platelet composition provided herein can also comprise adding to the suspension medium one or more salts, such as phosphate salts, sodium salts (e.g., NaCl), potassium salts (e.g., KCl), calcium salts, magnesium salts, and any other salt that can be found in blood or blood products, or that is known to be useful in cryopreserving platelets, or any combination of two or more of these.

In some embodiments, the salts are present in the composition at a concentration of about 1 mM to about 1000 mM, such as about 0.01 M to about 0.2 M. In some embodiments, one or more salts are present in about 0.4 to about 40 mg/ml, or about 0.4 to about 4 mg/ml, or about 4 mg/ml to about 40 mg/ml in the composition. In some embodiments, one or more salts are present in about 0.03 to about 3 mg/ml, or about 0.03 to about 0.3 mg/ml, or about 0.3 mg/ml to about 3 mg/ml in the composition.

In some embodiments, these salts are present in the composition at an amount that is about the same as is found in whole blood.

In some embodiments, the method for preparing a cryopreserved platelet composition includes adding to the suspension medium an organic solvent, such as an alcohol, such as ethanol, to the suspension medium. The organic solvent can include one or more alcohols, e.g., short-chain alcohols, such as ethanol. Short-chain alcohols are alcohols having 1 to 6 carbon atoms, in particular 2, 3 or 4 carbon atoms, such as methanol, ethanol, and propanol including 1-propanol and 2-propanol, preferably ethanol. The organic solvent may also be a mixture of different organic solvents. In such a suspension medium, the solvent can range from 0.1 % to 5.0 % (v/v). In some embodiments, one or more organic solvents are present in about 0.08% (v/v) to about 8% (v/v), or about 0.08% (v/v) to about 0.8% (v/v), or about 0.8% (v/v) to about 8% (v/v) in the composition. Thus, in one embodiment, the first mixture comprises platelets, a cryoproctectant comprising trehalose and polysucrose, and a solvent, such as ethanol.

The method for preparing a cryopreserved platelet composition can include transferring the first mixture, which can comprise the suspended platelets in the suspension medium, to a sealable container (e.g., a container closure system) before subjecting the platelets to a low temperature, and/or freezing the platelets. In some embodiments, the sealable container is a bag such as a fluorinated ethylene propylene (FEP) bag or a polyvinyl chloride (PVC) bag. In some embodiments, the sealable container is a borosilicate serum vial. The platelets and/or suspension medium can be placed (e.g., by aliquotting) in the container at a desired dose size. In some embodiments, the dose size of the platelets can range from about 1×10⁷ platelets to about 3×10¹¹ platelets , such as about 2 x 10⁹ platelets.

The method further includes subjecting the first mixture in sealable container to a low temperature that forms the cryopreserved platelet composition. In some embodiments, the temperature freezes the suspended platelets in the suspension medium. The suspended platelets and suspension medium is subjected to a low temperature of less than -1 °C (e.g., about or less than about -5 °C, about -10 °C, about -20 °C, about -30 °C, about -40 °C, about -50 °C, about -60 °C, about -70 °C, about -80 °C, or about of less than about -90 °C). In some embodiments, the first mixture is subjected to a temperature of about -70°C to about - 90 °, -50°C to about -70 °, -30°C to about -50 °, -10°C to about -30 °, or about 10°C to about -10 °C.). In some embodiments, the first mixture is subjected to a temperature of about -80°C.

The method for preparing the cryopreserved platelet composition for use by thawing the frozen suspended platelets in the suspension medium. In some embodiments, the thawing includes submersing the cryopreserved platelet composition in a 37°C water bath for a suitable amount of time, e.g., about 8 minutes to about 10 minutes.

The cryopreserved platelet composition is the composition obtained from subjecting a mixture containing platelets, or platelet-derived materials, to a temperature of less than -1°C. In some embodiments, the cryopreserved platelet composition is either partially or fully frozen composition.

In some of the embodiments wherein a cryopreserved platelet composition is obtained by a method disclosed herein, the composition obtained from the freezing steps is thawed to form the cryopreserved platelet composition. Thus, in such embodiments, the method further comprises rehydrating the composition obtained from the freezing steps, to form the cryopreserved platelet composition.

In some embodiments, the method may optionally further include incubating the re-suspended platelets. The step of incubating the platelets to load them with a cryoprotectant includes incubating the platelets for a time suitable for loading, as long as the time, taken in conjunction with the temperature, is sufficient for the cryoprotectant to come into contact with the platelets and, preferably, be incorporated, at least to some extent, into the platelets. In embodiments, incubation is carried out for about 1 minute to about 180 minutes or longer. In some embodiments, the re-suspended platelets are incubated in an air-permeable bag (e.g., FEP bag) in an air incubator at about 37°C for about 3 hours with gentle agitation.

The step of incubating the platelets to load them with a cryoprotectant includes incubating the platelets and the cryoprotectant at a temperature that, when selected in conjunction with the amount of time allotted for loading, is suitable for loading. In general, the composition is incubated at a temperature above freezing (e.g., >1°C) for at least a sufficient time for the cryoprotectant to come into contact with the platelets. In embodiments, incubation is conducted at 37°C. In certain embodiments, incubation is performed at 20°C to 42°C. For example, in embodiments, incubation is performed at 35°C to 40°C (e.g., 37°C) for 110 to 130 (e.g., 120) minutes.

The method of preparing a cryopreserved platelet composition can optionally include a protectant, such as a high molecular weight polymer, into the loading composition. By "high molecular weight" it is meant a polymer having an average molecular weight of about or above 70 kDa.

The composition comprises 3% to 10% w/v of a polysucrose polymer. Non-limiting examples of further polymeric cryoprotectants are polymers containing one or more monosaccharide units. Non-limiting examples are polysaccharides. Non-limiting examples are polymers derived from sucrose, epichlorohydrin, or combinations thereof. In some embodiments, the polymers are made by the copolymerization of sucrose and epichlorohydrin, such as a polysucrose (e.g., Ficoll^{®} 70 and Ficoll^{®} 400 provided by GE Healthcare Bioprocess, Uppsala, Sweden). Although any amount of high molecular weight polymer can be used, it is preferred that an amount be used that achieves a final concentration of about 3% to 10% (w/v), such as 3% to 7% (w/v), for example 6% (w/v). In some embodiments, one or more further polymers are present in about 6 mg/ml to about 600 mg/ml, or about 6 mg/ml to about 60 mg/ml, or about 60 mg/ml to about 600 mg/ml in the composition. Other non-limiting examples of lyoprotectants are serum albumin, dextran, polyvinyl pyrolidone (PVP), starch, and hydroxyethyl starch (HES). In some embodiments, a lyoprotectant provided herein may also, or alternatively, serve as a cryoprotectant.

The cryopreserved platelet composition provided herein is highly stable, having a shelf- life of at least six months or above, such as at least eighteen months, at room temperature or below. For example, the cryopreserved platelet composition is stable for at least six months at a temperature of about -80°C +/- 10°C. For example, the cryopreserved platelet composition can show hemostatic triggers for primary coagulation properties up to one year after manufacture when stored at room temperature or below, up to 18 months at room temperature or below, or even longer. By "stable" it is meant that the platelets of the cryopreserved platelet composition function within the parameters mentioned above, and provide adequate hemostatic triggers for primary coagulation. These clotting functions include hemostatic function and primary coagulopathic function. This stability is of great advantage in providing platelet products to those in need, particularly those found at sites some distance from blood collection centers, those in combat theaters, and those working in disaster areas as first responders. Furthermore, because the cryopreserved platelet composition can be stored at room temperature up to 40°C for long-term storage and up to 80°C to 90°C for short periods of about 24 hours, complicated, bulky, or expensive containers for storage (e.g., refrigerators) are not needed. That is, the problem of cold-chain storage is eliminated.

When needed for treatment of bleeding and for formation of clots, use as a primary hemostatic agent, or for research purposes, the cryopreserved platelet composition of this disclosure can be thawed. The thawed cryopreserved platelet composition is considered a liquid composition according to this disclosure. **In** some embodiments, the frozen compositions can be thawed to form a liquid composition at a temperature that is above 1°C. In some embodiments, the cryopreserved platelet composition is thawed at a temperature of about 37°C for about 8 to about 10 minutes.

As will be recognized by those of skill in the art, the cryopreserved platelet composition of this disclosure has the ability to act as a hemostatic agent to form clots at sites of injury involving bleeding. This concept can be understood as use of the composition for treating bleeding or a method for treating a subject having a bleeding wound. In general, the method comprises contacting a site of bleeding with a sufficient amount of a composition provided herein to reduce or stop the bleeding. The step of contacting can be performed in any suitable way, including, but not necessarily limited to, i) systemic administration of the composition via intravenous infusion or bolus injection and ii) topical administration directly to the site of bleeding. For intravenous administration, the composition is a liquid composition. It should be noted that intravenous administration is suitable for both treating bleeding due to a wound or other trauma and treating bleeding due to coagulopathy. For topical administration, the cryopreserved platelet composition is a liquid composition. When topically administering the liquid form, the composition can be dripped, sprayed, or poured (or the equivalent) directly onto the site of bleeding. The methods of treating, whether therapeutic or prophylactic, of this disclosure can further comprise administering a composition provided herein a second or multiple times. Therefore, the methods of this disclosure encompass treatment regimens in which administration is repeated one or more times at preselected time intervals. Successive administrations may include administration of additional components. The choice of amounts and composition components can be selected by those of skill in the art based on various parameters commonly considered by those of skill in the art, such as subject age, weight, health history, clinical presentation, ancillary presentations, and the like. It is well within the skill of those in the art to make appropriate changes and adjustments to treatment regimens without undue experimentation.

Certain embodiments of this disclosure provide frozen and liquid cryopreserved platelet compositions for the treatment of drug-induced coagulopathy and for the accelerated efficacy of procoagulant drugs. For example, the cryopreserved platelet compositions of the invention overcome the deficiencies seen in anticoagulant therapy subjects and other subjects showing delayed or absent clotting by providing at least one component in the clotting cascade that is downstream of the component that is lacking in these subjects.

The cryopreserved platelet composition provided herein is suitable for administering to a subject in an amount sufficient to raise the hemostatic or coagulation properties of that subject's blood to a level that is detectably higher than it was before administration. The method can further comprise administering other biologically active agents, such as clotting factors and/or chemotherapeutic agents for treatment of cancer.

The cryopreserved platelet composition provided herein is suitable for use in monitoring the progression of a disease or disorder of the blood clotting system. The methods generally comprise combining a composition provided herein with platelets and/or plasma removed from a subject suffering from the disease or disorder to make a mixture, and determining the blood clotting ability of the mixture. Typically, determining the blood clotting ability of the mixture indicates the blood clotting ability of the subject's blood, and comprises assaying clotting time of the mixture. Furthermore, typically, multiple assays are performed over time to give an indication of progression over time.

Herein are also disclosed kits. In general, a kit of this disclosure comprises any one of the cryopreserved platelet compositions provided herein. The container can be any material suitable for containing the composition, such as a vial, an ampule, or a bag. The container may comprise a sufficient amount of composition to perform at least one embodiment of at least one method provided herein. Thus, the kits can be, among other things, diagnostic kits, blood clotting monitoring kits for coagulation proteins or platelets, or drug treatment monitoring kits. The container may be provided as a component of a larger kit, which includes suitable packaging and, optionally, instructions and other information relating to use of the compositions. The container or kit may comprise other components, such as purified components of the clotting cascade. The kit can be configured to supply the composition for use in in vivo treatments, for use in in vitro diagnostics, or for use in in vitro or in vivo research. Often, the kits will comprise some or all of the supplies and reagents to perform one or more control reactions to ensure the kits are performing properly and to provide baseline results against which test samples can be compared. The composition may be provided in a sufficient amount to treat a subject in need of platelet function, such as a subject undergoing surgery or having a bleeding wound. A sufficient amount of the composition may be provided to perform studies on platelets or the blood clotting system of humans, or other animals.

### EXAMPLES

Herein are also disclosed kits. In general, a kit may comprise any one of the cryopreserved platelet compositions provided herein. The container can be any material suitable for containing the composition, such as a vial, an ampule, or a bag. The container may comprise a sufficient amount of composition. Thus, the kits can be, among other things, diagnostic kits, blood clotting monitoring kits for coagulation proteins or platelets, or drug treatment monitoring kits. The container may be provided as a component of a larger kit, which includes suitable packaging and, optionally, instructions and other information relating to use of the compositions. The container or kit may comprise other components, such as purified components of the clotting cascade. The kit can be configured to supply the composition for use in in vivo treatments, for use in in vitro diagnostics, or for use in in vitro or in vivo research. Often, the kits will comprise some or all of the supplies and reagents to perform one or more control reactions to ensure the kits are performing properly and to provide baseline results against which test samples can be compared. The composition may be provided in a sufficient amount to treat a subject in need of platelet function, such as a subject undergoing surgery or having a bleeding wound. A sufficient amount of the composition is provided to perform studies on platelets or the blood clotting system of humans, or other animals.

### Example 1: Platelet Function Metrics of Low DMSO and DMSO-Free Platelet Compositions

### A. Sample Preparation

Exemplary cryopreserved platelet compositions were prepared as described herein.

Platelets, contained in a liquid medium, obtained were untreated, human apheresis platelet material ("hIDSP"). The untreated, apheresis platelets were subjected to processing, including isolating platelets by applying centrifugation and tangential flow filtration. Centrifugation was carried out at ~1500 g for 20 minutes, with slow acceleration and braking. Following the centrifugation process, supernatant plasma from the apheresis platelets was aspirated and disposed. Tangential flow filtration was carried out with a 0.45µm filter for >3 diavolumes.

The liquid medium was replaced with a buffer Formulation A or Formulation B *(see* Tables 1 and 2, respectively) to form exemplary platelet compositions "Std LB" and "Std LB w/ 1% DMSO." The platelet composition (Std LB w/ 1% DMSO) in Formulation A contained DMSO in an amount of 1.0% (v/v). The platelet composition ("Std LB") in Formulation B contained no DMSO. Each of the platelet compositions contained a concentration of 2×10⁹ platelets/ml.

The platelet composition was incubated in an air-permeable bag, made of FEP material, in an air incubator at 37°C for 3 hours with gentle agitation.

Following incubation, the platelet composition was transferred to a bag appropriate for freezing. The platelets were aliquoted into the bag in a platelet dose size of about 3 x 10¹¹ platelets per bag.

**TABLE 1: Components of Buffer Formulation A**

| **Excipient** | **Concentration** |
|---|---|
| **NaCl** | 3.51 mg/ml |
| **Kcl** | 0.29 mg/ml |
| **HEPES** | 1.81 mg/ml |
| **NaHCO3** | 0.81 mg/ml |
| **Dextrose** | 0.43 mg/ml |
| **Trehalose** | 30.27 mg/ml |
| **Ficoll PM 400 Polysucrose** | 60.00 mg/ml |
| **Ethanol** | 0. 8% (v/v) |
| **DMSO** | 1.0% (v/v) |
| **Water** | Balance |

**TABLE 2: Components of Buffer Formulation B**

| **Excipient** | **Concentration** |
|---|---|
| **NaCl** | 3.51 mg/ml |
| **Kcl** | 0.29 mg/ml |
| **HEPES** | 1.81 mg/ml |
| **NaHCO3** | 0.81 mg/ml |
| **Dextrose** | 0.43 mg/ml |
| **Trehalose** | 30.27 mg/ml |
| **Ficoll PM 400 Polysucrose** | 60.00 mg/ml |
| **Ethanol** | 0.8%(v/v) |
| **Water** | Balance |

The bags containing the platelet compositions (Std LB, Std LB w/ 1% DMSO) and untreated platelets (hIDSP Thawed) were loaded into freezer at a temperature of -80°C to form cryopreserved platelet compositions. The bags were loaded directly onto the freezer shelf such that the bags made full contact with the shelf and were allowed to equilibrate for >24 hours.

Prior to testing, the cryopreserved platelet compositions were thawed in a 37°C water bath for 8-10 minutes.

### B. Platelet Count

The platelet compositions suspended in buffer Formulation A (with DMSO) or Formulation B (without DMSO) were tested for platelet count (AcT Diff Count) using an automated Coulter AcT Diff system. These platelet compositions were compared against the untreated platelet ("hlDSP").

**TABLE 3: Platelet AcT Count**

| **Product** | **AcT Count (×10³ platelets/µl)** | | |
|---|---|---|---|
| | Post-Processing | Thawed | Yield |
| **hIDSP** | 1613 | 1360 | 84% |
| **Std LB** | 1677 | 1537 | 92% |
| **Std LB w/ 1% DMSO** | 1735 | 1680 | 97% |

Table 3 provides platelet counts obtained using the AcT Diff cell counter for the untreated platelets (hIDSP), the platelet composition without DMSO ("Std LB"), and platelet composition 1% DMSO ("Std LB w/ 1% DMSO") before freezing ("Post-Processing"), and after being thawing ("Thawed"). "Post-processing" refers to platelets and platelet compositions that have been subjected to processing associated with isolating platelets through centrifugation and/or tangential flow filtration.

The untreated platelets (hIDSP) yielded the lowest platelet count after being thawed. Buffer formulation A (Std LB w/ 1% DMSO) had >90% platelet recovery after thawing.

### C. Particle Size Distribution By Flow Cytometry Forward Scattering

Flow Cytometry Forward Scattering was performed on the samples at both the post-processing and thawed states to determine their size distributions. The tested samples included the (i) untreated platelets (hIDSP); (ii) the untreated apheresis platelets (hIDSP Thawed); the exemplary DMSO-free platelet composition (Std LB), and (iii) the exemplary platelet composition with 1% DMSO (Std LB w/ 1% DMSO).

FIG. 1 provides a bar graph showing the platelet size distribution of (i) untreated platelets, (ii) thawed untreated platelets, (iii) the composition without DMSO; and (iv) the composition with 1 % (v/v) DMSO.

The data demonstrated that the untreated platelets after thawing ("hIDSP Thawed") exhibited the largest size deviation, as compared to the initial platelet size distribution ("hIDSP"). The data demonstrated that the cryopreserved compositions, after being thawed, exhibited similar results, when compared to the platelet size distribution of the untreated platelets (hIDSP). The platelet composition with 1% DMSO (Std LB w/ 1% DMSO) appeared to exhibit platelet size distributions that were most similar to the untreated platelets (hIDSP).

### D. Particle Size Distribution By Thrombolux Dynamic Light Scattering

Thrombolux dynamic light scattering testing was conducted to determine size distributions for the untreated platelets (hIDSP), the thawed, untreated apheresis platelets ("hIDSP Thawed"), and the thawed cryopreserved platelet compositions with 1% DMSO ("Std LB w/ 1% DMSO").

FIG. 2 provides a graph plot of Thrombolux dynamic light scattering data showing the size distributions the different test groups. The size distribution data, as shown, were consistent with the data obtained from the flow cytometry testing *(see* Section B, *supra).* The thawed hIDSP group showed the largest size deviation as compared to the initial platelets, and also showed a large microparticle population. The platelet composition with 1% DMSO ("Std LB w/ 1% DMSO") was similar to the initial platelets size distribution. Furthermore, bimodal peaks in the size distribution data of the "hIDSP Thawed" group indicated a significant amount of microparticle development. The size distribution of the exemplary platelet compositions with 1% DMSO ("Std LB w/ 1% DMSO"), however, were similar to the initial platelet size distribution ("hIDSP").

**TABLE 4: Thrombolux Dynamic Light Scattering Test Data**

| **Product** | **Blue intensity [%]** | **Blue peak radius [nm]** | **Blue PDI** | **Cyan intensity [%]** | **Cyanpeak radius [nm]** |
|---|---|---|---|---|---|
| **hIDSP** | 94% | 1455 | 0.1 | 6% | 52 |
| **hIDSP Thawed** | 69% | 1045 | 0.5 | 28% | 93 |
| **Std LB** | 97% | 937 | 0.5 | 2% | 52 |
| **Std LB w/ 1% DMSO** | 98% | 1109 | 0.2 | 2% | 46 |

Table 4 provides the Thrombolux size data obtained for the untreated platelets (hIDSP) and the thawed cryopreserved platelet formulations. "Blue intensity" represents the percent of measured particles that fall within the platelet size region; "Blue peak radius" gives the mean size of that population; and "Blue PDI" gives the polydispersity, or spread, of the population. The "Cyan intensity" represents the percent of measured particles that fall below the platelet size; and "Cyan peak radius" provides the mean size of that population.

The data in Table 4 demonstrated the thawed hIDSP showed the most significant size differences, as compared to the initial platelets, with only 69% of the particles falling in the platelet region (vs. an initial 94%) and a significantly smaller mean radius. The other thawed formulations were similar their size distributions to the initial platelets. The exemplary platelet composition with 1% DMSO ("Std LB w/ 1% DMSO") was the most similar to the untreated platelet size distribution. The exemplary platelet composition with 1% DMSO had the greatest proportion of platelet sized particles and a smaller Blue PDI than the other compositions.

### E. Platelet Aggregation Testing

The untreated platelets and platelet compositions were subjected to aggregometry testing. Platelet aggregation is a fundamental hemostatic function that results from platelet activation by various agonists. In fresh platelets, aggregation can be used to screen for functional activation and adhesion receptors on the platelet surface.

In turbidimetric aggregometry, light is passed through a stirred suspension of platelets. A certain degree of light is scattered by the platelets, which reduces the amount of light passing through the sample. This allows the amount of light that passes through the sample, when measured, to provide an optical density measurement associated with the sample. A high optical density (i.e., low light transmission) indicates the presence of many individual platelets. Aggregates form when the platelets clump together. Aggregates generally reduce the amount of light that is scattered by the platelets, such that, in some cases, the light can pass mostly unobstructed through the suspension. This causes a decrease in the optical density of the sample, and yields a high light transmission. The percent of light transmission relative to a blank (solution without platelets) is reported as the percent aggregation.

In the Platelet Aggregation Test, the samples were prepared by diluting the thawed platelet compositions in HEPES Modified Tyrodes Albumin Buffer (HMTA). The proportion of thawed platelet compositions was 16% (v/v), having a count of about 250-300,000 platelets per µL ± 50,000 platelets per µL. The diluted samples were allowed to incubate in glass cuvettes with magnetic stir bars at 37°C with stirring for >2 minutes. The optical density (light transmission) of each sample was then measured continuously using a Helena AggRAM machine with HemoRAM software (version 1.3). After 2 minutes, in which a baseline was established, various agonists were added to induce aggregation. Agonists used included: 2.5 units of thrombin, 10 µg/ml of collagen, 50 µg/ml of arachidonic acid, or 20 µM Thrombin Receptor Activating Peptide (TRAP-6). Samples were measured for about 10 minutes, and the highest percent aggregation during the 10 minutes of measurement was reported in the aggregation test results.

FIG. 3 provides the aggregation test results for the untreated platelets (hIDSP) and the thawed cryopreserved exemplary platelet composition with 1% DMSO ("Std LB w/ 1% DMSO"), and the thawed, untreated platelets ("hIDSP Thawed").

The test results showed that the thawed, untreated platelets (hIDSP Thawed) provided the lowest response in every category and was most different from the untreated platelets (hIDSP). The platelet composition with 1% DMSO ("Std LB w/ 1% DMSO") gave the highest response of the thawed products and was most similar to the untreated platelets (hIDSP).

### F. Summary

Based on the test results provided in Sections B-E (*supra*), the compositions containing DMSO exhibited more desirable platelet function, based on the size distribution, yield, and aggregation test data, than the other test groups (hIDSP and hIDSP Thawed).

### Example 2: Platelet Function Metrics of DMSO-Free Platelet Compositions

This example provides data of platelet function (e.g., size distribution, yield, and aggregation characteristics) of exemplary compositions containing no DMSO when subjected to different low temperature conditions.

### A. Sample Preparation

Exemplary platelet compositions with no DMSO are prepared as described below.

Platelets contained in a liquid medium were isolated by applying centrifugation filtration. The liquid medium was replaced with the buffer Formulation B (see Table 2), such that the platelets were suspended in the buffer Formulation B.

The platelet composition were subjected to different low temperature for 12 hours to form the cryopreserved platelets. The different temperatures included: (i) - 80°C following a gradual -1°C/minute temperature rate of adjustment to -70°C ("Thawed"), (ii) -80°C ("-80C Thawed"), or (iii) -20°C ("-20C Thawed"). The -80C Thawed and -20C Thawed samples were placed immediately in the low temperature condition without any gradual temperature adjustment.

After the freezing process, the platelet compositions were stored at -80C for at least 24 hours prior to testing.

Prior to testing, the cryopreserved platelet compositions were thawed in a 37°C water bath.

### B. Platelet Count

The DMSO-free platelet compositions suspended in Buffer Formulation B were tested for a platelet count (AcT Diff Counts) using an automated Coulter AcT Diff system.

**TABLE 5: Platelet AcT Count**

| **Product** | **AcT Count (×10³ platelets/µl)** | | |
|---|---|---|---|
| | Post-Processing | Thawed | Yield |
| **Thawed** | 1677 | 1625 | 97% |
| **-80C Thawed** | 1677 | 1675 | 100% |
| **-20C Thawed** | 1677 | 1300 | 78% |

Table 5 provides platelet counts by AcT Diff cell counter of the exemplary compositions that were subject to the different temperature conditions.

The platelet composition subjected to -20°C (-20C Thawed) exhibited the lowest platelet yield after thawing. The remaining compositions (Thawed, -80C Thawed) were comparable close in yield, each having a >90% platelet recovery after thawing.

### C. Particle Size Distribution By Flow Cytometry Forward Scattering

Flow Cytometry Forward Scattering was performed on the exemplary DMSO-free platelet composition (i) prior to freezing (Pretreat); and on the thawed exemplary DMSO-free platelet compositions that were frozen at (ii) -80°C following a -1°C/minute temperature adjustment to -70°C (Thawed), (iii) -80°C (-80C Thawed), or (iv) -20°C (-20C Thawed).

FIG. 4 provides flow cytometry forward scattering data for a platelet size distribution of each of the DMSO-free platelet compositions.

The exemplary platelet composition that was subjected to a temperature - 20°C (-20C Thawed) exhibited the largest size deviation, as compared to the initial platelet size distribution (Pretreat). The -20°C Thawed platelet composition had a greater proportion of micro-particles than any of the other test groups (Pretreat, Thawed, -80C Thawed).

### D. Particle Size Distribution By Thrombolux Dynamic Light Scattering

Thrombolux dynamic light scattering testing determines size distributions for the DMSO-free platelet compositions (i) prior to freezing (Pretreat); and for the thawed exemplary DMSO-free platelet compositions that were frozen at (ii) -80°C following a -1°C/minute temperature adjustment to -70°C (Thawed), (iii) -80°C (-80C Thawed), or (iv) -20°C (-20C Thawed).

FIG. 5 provides a graph plot of Thrombolux dynamic light scattering data showing the size distributions of the different test groups. The size distribution data, as shown, is consistent with the data obtained from the flow cytometry testing *(see* Section B, *supra).* The platelet composition that was frozen at 20°C (-20C Thawed) shows the largest size deviation as compared to the initial platelets (Pretreat) and has a large microparticle population. The other platelet compositions (Thawed, -80C Thawed) exhibit similar size distributions as compared to the initial platelets (Pretreat).

**TABLE 6: Thrombolux Dynamic Light Scattering Test Data**

| **Product** | **Blue intensity [%]** | **Blue peak radius [nm]** | **Blue PDI** | **Cyan intensity [%]** | **Cyan peak radius [nm]** |
|---|---|---|---|---|---|
| **Pretreat** | 100% | 1198 | 0.3 | 0% | 10 |
| **Thawed** | 85% | 1158 | 0.5 | 14% | 210 |
| **-80C Thawed** | 91% | 1101 | 0.5 | 8% | 118 |
| **-20C Thawed** | 80% | 847 | 0.5 | 19% | 132 |

Table 6 provides the Thrombolux size data for the exemplary DMSO-free platelet composition (i) prior to freezing (Pretreat); and on the thawed exemplary DMSO-free platelet compositions that were frozen at (ii) -80°C following a - 1°C/minute temperature adjustment to -70°C (Thawed), (iii) -80°C (-80C Thawed), or (iv) -20°C (-20C Thawed). As discussed previously, "Blue intensity" represents the percent of measured particles that fall within the platelet size region; "Blue peak radius" gives the mean size of that population; and "Blue PDI" gives the polydispersity, or spread, of the population. The "Cyan intensity" represents the percent of measured particles that fall below the platelet size; and "Cyan peak radius" provides the mean size of that population.

As shown in Table 6, the platelet composition that was frozen at 20°C (-20C Thawed) exhibited the most significant size difference, as compared to the initial platelets (Pretreat). Only 80% of the particles were in the platelet region (vs. an initial 100%), and the particles had a significantly smaller mean radius. The other platelet compositions subjected to different freezing conditions (-80C Thawed, Thawed) exhibit results similar to the initial platelets (Pretreat).

### E. Platelet Aggregation Testing

Aggregometry testing was performed on the exemplary DMSO-free platelet composition (i) prior to freezing (Pretreat); and on the thawed exemplary DMSO-free platelet compositions that were frozen at (ii) -80°C following a - 1°C/minute temperature adjustment to -70°C (Thawed), (iii) -80°C (-80C Thawed), or (iv) -20°C (-20C Thawed).

FIG. 6 provides the aggregation test results of each of the exemplary platelet compositions.

The platelet composition that was frozen at 20°C (-20C Thawed) exhibited the lowest response in every category (thrombin, collagen, and arachidonic acid) and deviated the most from the initial platelets (Pretreat). The remaining platelet compositions (-80C Thawed, Thawed) exhibited similar results as the initial platelets

(Pretreat) when responding to thrombin, but exhibited a lower response as compared to the initial platelets (Pretreat) when responding to collagen and arachidonic acid.

### F. Summary

Gradual and rapid freezing of the exemplary DMSO-free platelet compositions at -80°C yielded better platelet function as measured by metrics: size distribution, yield, and aggregation, as compared to the rapid freezing of DMSO-free platelet compositions at -20°C.

### Example 3: Platelet Function Metrics of DMSO-Free Platelet Compositions

This example provides exemplary compositions containing no DMSO when subjected to a low temperature condition in different sealed containers.

### A. Sample Preparation

Exemplary platelet compositions were prepared as described below. Platelets contained in a liquid medium were isolated by applying centrifugation filtration. The liquid medium was replaced with buffer Formulation B (see Table 2), and platelets were suspended in the buffer Formulation B. The exemplary platelet compositions with Formulation B contained no DMSO.

The platelet compositions were frozen at -80°C in fluorinated ethylene propylene (FEP) bags, polyvinyl chloride (PVC) bags, or in borosilicate serum vials to form cryopreserved platelet compositions.

Prior to testing, the cryopreserved platelet compositions were thawed in a 37°C water bath.

### B. Platelet Count

Each of the platelet compositions frozen in a FEP bag, a PVC bag, or a borosilicate vial were tested for platelet count (AcT Diff Counts) using an automated Coulter AcT Diff system.

**TABLE 7: Platelet AcT Count**

| | AcT Count (×10^3 platelets/µl) | | |
|---|---|---|---|
| Product | Post-Processing | Thawed | Yield |
| Prelyo | 1850 | - | - |
| -80C St Gobain Thawed | 1850 | 1883 | 102% |
| -80C Terumo Thawed | 1850 | 1857 | 100% |
| -80C Vial Thawed | 1850 | 1867 | 101% |

In table 7, "St Gobain" and "Terumo" refer to the type of container, such as a bag or vial, used to store the samples at the desired temperature. Examples are described at https://www.terumobct.com/blood-center-services/blood-bags , and at https://cellgenix.com/wp-content/uploads/2017/109/Kryosure_Kryovue_Cryopreservation_Brochure_CGX.pdf, both of which are incorporated by reference herein in their entirety.

Table 7 provides the platelet counts obtained by the AcT Diff cell counter for the exemplary compositions frozen in the different sealed containers (the FEP bag, the PVC bag, or the borosilicate vial).

The different sealed containers provided very similar yields, which were nearly identical to the pre-freezing ("post-processing") yields.

### C. Particle Size Distribution By Flow Cytometry Forward Scattering

Flow Cytometry Forward Scattering was performed on the platelet compositions prior to freezing ("Pretreat") and for each of the platelet compositions frozen in the FEP bag, the PVC bag, or the borosilicate vial.

As shown in FIG. 7, platelet compositions in the different product containers exhibited similar size distributions.

### D. Particle Size Distribution By Thrombolux Dynamic Light Scattering

Thrombolux dynamic light scattering testing determined the particle size distributions for the formulated product prior to freezing ("Pretreat") and for product frozen in the FEP bag, the PVC bag, or the borosilicate vial.

FIG. 8 provides a graph plot of Thrombolux dynamic light scattering data

**TABLE 8: Thrombolux Dynamic Light Scattering Test Data**

| **Product** | **Blue intensity [%]** | **Blue peak radius [nm]** | **Blue PDI** | **Cyan intensity [%]** | **Cyan peak radius [nm]** |
|---|---|---|---|---|---|
| **Pretreat** | 99% | 1312 | 0.3 | 1% | 21 |
| **FEP Bag** | 95% | 1288 | 0.4 | 4% | 105 |
| **PVC Bag** | 94% | 1231 | 0.4 | 5% | 118 |
| **Borosilicate Vial** | 94% | 1225 | 0.5 | 5% | 93 |

showing the size distributions of the different test groups. The size distribution data, as shown, was consistent with the flow cytometry data *(see* Section B, *supra).* All of the compositions were similar in size distribution.

Table 8 provides the Thrombolux size data for the exemplary platelet compositions prior to freezing ("Pretreat") and for product frozen in the FEP bag, the PVC bag, or the borosilicate vial. As discussed previously, "Blue intensity" represents the percent of measured particles that fall within the platelet size region; "Blue peak radius" gives the mean size of that population; and "Blue PDI" gives the polydispersity, or spread, of the population. The "Cyan intensity" represents the percent of measured particles that fall below the platelet size; and "Cyan peak radius" provides the mean size of that population.

The results showed that the different containers had a minimal effect on platelet particle size.

### E. Platelet Aggregation Testing

The platelet and platelet compositions were subjected to aggregometry testing.

FIG. 9 provides the aggregation test results for the exemplary platelet compositions prior to freezing ("Pretreat") and for product frozen in an FEP bag, PVC bag, or borosilicate vial.

The results showed that the different sealed containers did not impact the aggregation response of the final product.

### F. Summary

The type of sealed container used for containing compositions during freezing did not significantly impact the characteristics of the exemplary DMSO-free platelet compositions.

### Example 4: Platelet Function Metrics of Platelet Compositions Containing Low Amounts of DMSO and/or Glycerol

### A. Sample Preparation

Exemplary cryopreserved platelet compositions were prepared as described herein.

Platelets, contained in a liquid medium, obtained were untreated, human apheresis platelet material ("hIDSP"). For all tested compositions, the apheresis platelets were isolated by applying centrifugation and tangential flow filtration. Centrifugation was carried out at ~1500 g for 20 minutes, with slow acceleration and braking. Following the centrifugation process, supernatant plasma from the apheresis platelets was aspirated and disposed. Tangential flow filtration was carried out with a 0.45µm filter for >3 diavolumes.

For the cryopreserved platelet compositions, the original liquid medium was replaced with a buffer Formulations A and B *(see* Example 1, Tables 1 and 2 of Section A, *supra),* Formulation C *(see* Table 9 below), or Formulation D *(see* Table 10 below) to form the following exemplary platelet compositions:
a) **"hIDSP Thawed"** - A platelet composition containing isolated, untreated platelets. This composition serves as a control in this experiment.
b) **"Std LB"** - A platelet composition with Formulation B, which contains no DMSO.
c) **"LB + 1% DMSO"** - A platelet composition with Formulation A, which contains DMSO, 1.0% (v/v).
d) **"LB + 1% DMSO/Glycerol"** - A platelet composition ("LB+1% DMSO/Glycerol") with Formulation C, which contains DMSO, 1.0% (v/v) and glycerol, 1.0% (v/v).
e) **"LB + 1% Glycerol"** - A platelet composition ("LB+1% Glycerol") with Formulation D, which contains glycerol, 1.0% (v/v).

**TABLE 9: Components of Buffer Formulation C**

| **Excipient** | **Concentration** |
|---|---|
| **NaCl** | 3.51 mg/ml |
| **Kcl** | 0.29 mg/ml |
| **HEPES** | 1.81 mg/ml |
| **NaHCO3** | 0.81 mg/ml |
| **Dextrose** | 0.43 mg/ml |
| **Trehalose** | 30.27 mg/ml |
| **Ficoll PM 400 Polysucrose** | 60.00 mg/ml |
| **Ethanol** | 0.8% (v/v) |
| **DMSO** | 1.0% (v/v) |
| **Glycerol** | 1.0% (v/v) |
| **Water** | Balance |

**TABLE 10: Components of Buffer Formulation D**

| **Excipient** | **Concentration** |
|---|---|
| **NaCl** | 3.51 mg/ml |
| **Kcl** | 0.29 mg/ml |
| **HEPES** | 1.81 mg/ml |
| **NaHCO3** | 0.81 mg/ml |
| **Dextrose** | 0.43 mg/ml |
| **Trehalose** | 30.27 mg/ml |
| **Ficoll PM 400** | 60.00 mg/ml |
| **Polysucrose** | |
| **Ethanol** | 0.8% (v/v) |
| **Glycerol** | 1.0% (v/v) |
| **Water** | Balance |

Each of the platelet compositions contained a concentration of 2×10⁹ platelets/ml.

Each of the platelet compositions, with exception of the untreated platelets (hIDSP), were incubated during recirculation in plastic bottles and tubing at room temperature.

Following incubation, the platelet compositions (except the untreated platelets (hIDSP)) were transferred to a bag appropriate for freezing. The platelets compositions were aliquoted into each bag such that the platelet dose size was about 3 × 10¹¹ platelets per bag.

The bags containing each of the platelet compositions, with exception of the untreated platelets (hIDSP), were loaded into freezer at a temperature of -80°C. The bags were loaded directly onto the freezer shelf such that the bags made full contact with the shelf and were allowed to equilibrate for >24 hours.

Prior to testing, the platelet compositions, except for the untreated platelets (hIDSP), were thawed in a 37°C water bath for 8-10 minutes.

### B. Platelet Count

The untreated platelets and the cryopreserved platelet compositions were tested for platelet count (AcT Diff Count) using an automated Coulter AcT Diff system.

**TABLE 11: Platelet AcT Count**

| | **AcT Count (×10³ platelets/µl)** | | |
|---|---|---|---|
| **Product** | Post-Processing | Thawed | Yield |
| **Std LB** | 1930 | 1837 | 95% |
| **LB + 1% DMSO** | 1997 | 1827 | 91% |
| **LB + 1% Glycerol** | 1957 | 1830 | 94% |
| **LB + 1% DMSO/Glycerol** | 1920 | 1890 | 98% |

Table 11 provides platelet counts obtained using the AcT Diff cell counter for each of the exemplary platelet compositions of isolated platelets before freezing ("Post-Processing") and after being thawing ("Thawed").

The platelet compositions containing buffer Formulation A (w/ 1% DMSO), Formulation C (w/ 1% DMSO and glycerol), and Formulation D (w/ 1% glycerol), each had >90% platelet recovery after thawing, with the platelet composition containing buffer Formulation C having the highest platelet recovery (98%).

### C. Particle Size Distribution By Flow Cytometry Forward Scattering

Flow Cytometry Forward Scattering was performed on exemplary platelet compositions to determine platelet size distributions. The tested compositions included the (i) untreated platelets (hIDSP); and the cryopreserved platelet compositions, including: (ii) the DMSO-free platelet composition (Std LB); (iii) the platelet composition with 1% DMSO (LB + 1% DMSO); (iv) the platelet composition with 1% DMSO and 1% glycerol (LB + 1% DMSO/Glycerol); and (v) the platelet composition with 1% glycerol (LB + 1% glycerol).

FIG. 10 provides a bar graph showing the platelet size distribution of each of the exemplary platelet compositions.

The data demonstrated that the cryopreserved compositions, after being thawed, generally exhibited similar results to another, when compared to the initial platelet size distribution (hIDSP). The platelet compositions containing 1% DMSO (LB + 1% DMSO, LB + 1% DMSO/Glycerol) showed platelet size distributions that were slightly more similar to the untreated platelets (hIDSP).

### D. Particle Size Distribution By Thrombolux Dynamic Light Scattering

Thrombolux dynamic light scattering testing was conducted to determine size distributions for the untreated platelets (hIDSP), and each of the cryopreserved platelet compositions.

FIG. 11 provides a graph plot of Thrombolux dynamic light scattering data showing the size distributions the different compositions.
1. The size distribution data using the Thrombolux dynamic light scattering showed that each of the cryopreserved groups exhibited similar platelet size distributions.

**TABLE 12: Thrombolux Dynamic Light Scattering Test Data**

| **Product** | **Blue intensity [%]** | **Blue peak radius [nm]** | **Blue PDI** | **Cyan intensity [%]** | **Cyan peak radius [nm]** |
|---|---|---|---|---|---|
| **hIDSP** | 98% | 1285 | 0.2 | 2% | 52 |
| **Std LB** | 97% | 1160 | 0.5 | 3% | 83 |
| **LB + 1% DMSO** | 98% | 990 | 0.4 | 1% | 52 |
| **LB + 1% glycerol** | 97% | 829 | 0.4 | 2% | 52 |
| **LB w/ 1% DMSO/glycerol** | 98% | 1017 | 0.4 | 2% | 59 |

Table 12 provides the Thrombolux size data obtained for the untreated platelets (hIDSP) and the thawed cryopreserved platelet formulations. "Blue intensity" represents the percent of measured particles that fall within the platelet size region; "Blue peak radius" gives the mean size of that population; and "Blue PDI" gives the polydispersity, or spread, of the population. The "Cyan intensity" represents the percent of measured particles that fall below the platelet size; and "Cyan peak radius" provides the mean size of that population.

The results provided in Table 12 demonstrated that each of the cryopreserved platelet compositions exhibited similar size distribution data.

It can be noted that the size distribution data, as provided in FIG. 11 and Table 12, was not fully consistent with the data obtained from the flow cytometry testing (shown in FIG. 10 in Section B, *supra).* Slight differences between the flow cytometry testing and the Thrombolux dynamic light scattering testing were likely due to changes in the viscosity of the compositions induced by the addition of glycerol and DMSO to some of the formulations.

### E. Activation Assessment (CD62P Expression)

The untreated platelets and cryopreserved platelet compositions were subjected to a platelet activation test to determine whether the compositions showed observable reactivity to a CD62P antibody, when using flow cytometry. In particular, the platelet compositions were observed for their reactivity to the CD62P antibody when assayed by fluorescence using a BD Accuri C6 Plus, using BD Accuri C6 Plus Software version 1.0.23.1.

FIG. 12 is a flow cytometry data of an exemplary cryopreserved platelet compositions showing detected observable reactivity of the platelets to CD62P, which is a mouse anti-human antibody conjugated to *adenomatous polyposis coli* (APC).

The results indicate that each of the cryopreserved platelet compositions exhibited a similar percent of activation for CD62P expression. Thus the presence of cryoprotectants such DMSO and/or glycerol do not affect the platelet activation percentage of CD62P expression.

### F. Platelet Aggregation Testing

The untreated platelets and cryopreserved platelet compositions were subjected to aggregometry testing, as described in previous sections (*see e.g.,* Example 1, Section E), when using thrombin, collagen, or arachidonic acid as stimulating substances to induce the platelet aggregation.

FIG. 13 provides the aggregation test results for the untreated platelets (hIDSP) and the cryopreserved platelet compositions.

The test results showed no significant difference in percent aggregation in thrombin between the different cryopreserved platelet compositions. The test results, however, showed that platelet composition containing no DMSO (Std LB) exhibited the least amount of percent aggregation in collagen and arachidonic acid, as compared to the other cryopreserved platelet compositions, which contained DMSO (only), glycerol (only) or both DMSO and glycerol. The platelet compositions containing DMSO (LB + 1% DMSO, LB + 1% DMSO /glycerol) exhibited the highest percent of platelet aggregation by collagen and arachidonic acid.

### G. Summary

Based on the results provided in Sections B-F (*supra*), the composition containing DMSO with glycerol (LB + 1% DMSO/glycerol), exhibited slightly better platelet function, based on the size distribution, yield, and aggregation test data, as compared to untreated platelets (hIDSP) and all of the other platelet compositions (STD LB, LB + 1% DMSO, LB + 1% glycerol). The composition containing DMSO only (LB + 1% DMSO) performed slightly better than the composition containing glycerol only (LB + 1% glycerol) and the standard loading buffer (STD LB). The composition containing glycerol only (LB + 1% glycerol) exhibited size distribution and platelet yields that were most similar to the standard loading buffer (STD LB).

While the embodiments of the invention are amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

## Claims

1. A composition comprising:
cryopreserved platelets, wherein the cryopreserved platelets are a frozen platelet suspension; and
a cryoprotectant comprising dimethyl sulfoxide (DMSO) in an amount of less than 6% v/v in the composition, wherein the cryoprotectant further comprises a combination of a polysaccharide and a disaccharide, wherein the polysaccharide is a polysucrose polymer having a concentration in the range of 3% to 10% w/v in the composition, and wherein the disaccharide is trehalose in an amount in the range of 1.5% to 5% w/v in the composition, and wherein the composition is not obtained using a lyophilization step.

2. The composition of claim 1, wherein the DMSO is present in the amount of 0.5% v/v to 1% v/v or 1% v/v to 3% v/v.

3. The composition of claim 1, wherein the composition further comprises a buffering component.

4. The composition of claim 1, wherein the polysucrose and trehalose are present in the composition at a ratio of 1:1 to 5:1.

5. The composition of claim 3, wherein the buffering component is selected from the group consisting of phosphate buffered saline (PBS), bicarbonate/carbonic acid, such as a sodium-bicarbonate buffer, N-2-hydroxyethylpiperazine-N'-2- ethanesulfonic acid (HEPES), a tris-based buffer, a tris-buffered saline (TBS), and combinations thereof, wherein the composition further comprises a salt selected from the group consisting of a phosphate salt, a sodium salt, a potassium salt, a calcium salt, a magnesium salt, and combinations thereof; and wherein the composition is a hemostatic composition.

6. The composition of claim 1, wherein the DMSO is present in the amount of 0.5% v/v to 2% v/v in the composition.

7. A method of making a cryopreserved platelet composition, comprising:
obtaining platelets contained in a liquid medium;
suspending the platelets in a suspension medium comprising a cryoprotectant comprising DMSO in an amount of less than 6% v/v in the composition, wherein the cryoprotectant further comprises a combination of a polysaccharide and a disaccharide, wherein the polysaccharide is a polysucrose polymer present at 3% to 10% w/v, and wherein the disaccharide is trehalose present at 1.5% to 5% w/v to load the platelets with the cryoprotectant to form a cryoprotectant-loaded platelet suspension; and
subjecting the cryoprotectant-loaded platelet suspension to a temperature of less than - 1°C, for a time sufficient to freeze the cryoprotectant-loaded platelet suspension, thereby forming the cryopreserved platelet composition, wherein the method does not comprise a lyophilization step.

8. The method of claim 7, wherein the DMSO is in the amount of less than 1% v/v or from 1% v/v to 3% v/v.

9. The method of claim 7, wherein the method further comprises storing the platelets for at least 6 months at a temperature of -80 °C +/- 10 °C, wherein after the storing the cryopreserved platelet composition provides adequate hemostatic function and primary coagulopathic function.

10. The method of claim 7, wherein the suspension medium further comprises an organic solvent, wherein the organic solvent is present in an amount of 0.8% to 8% v/v of the suspension medium, wherein the organic solvent comprises a short-chain alcohol selected from the group consisting of methanol, ethanol, propanol, 1-propanol, 2-propanol, and combinations thereof.

11. The method of any one of claims 7 to 10, wherein the suspension medium has a pH of 4.0 to 7.0.

12. The method of any one of claims 7 to 10, wherein the suspension medium has a pH of 6.2 to 8.0, and wherein the suspending the platelets in the suspension medium is done at a temperature of 20°C to 42°C.

13. The composition of any one of claims 1 to 6, wherein the cryopreserved platelets comprise platelets derived from a human.

14. The composition of any one of claims 1 to 6, or 13, wherein at least 50% of the cryopreserved platelets are in the size range of 0.3µm to 5.0µm.

15. The composition of any one of claims 1 to 6, or 13, wherein the cryoprotectant further comprises glycerol in an amount of 0.1% v/v to 10% v/v in the composition.

## Patentansprüche

1. Zusammensetzung, umfassend:
kryokonservierte Thrombozyten, wobei die kryokonservierten Thrombozyten eine gefrorene Thrombozytensuspension sind; und
ein Kälteschutzmittel, umfassend Dimethylsulfoxid (DMSO) in einer Menge von weniger als 6 % Vol./Vol. in der Zusammensetzung, wobei das Kälteschutzmittel ferner eine Kombination aus einem Polysaccharid und einem Disaccharid umfasst, wobei das Polysaccharid ein Polysucrosepolymer mit einer Konzentration im Bereich von 3 % bis 10 % Vol. in der Zusammensetzung ist, und wobei das Disaccharid Trehalose in einer Menge im Bereich von 1,5 % bis 5 % Gew./Vol. in der Zusammensetzung ist, und wobei die Zusammensetzung nicht durch einen Gefriertrocknungsschritt erhalten wird.

2. Zusammensetzung nach Anspruch 1, wobei das DMSO in einer Menge von 0,5 % Vol. bis 1 % Vol. oder 1 % Vol. bis 3 % Vol. vorhanden ist.

3. Zusammensetzung nach Anspruch 1, wobei die Zusammensetzung ferner eine puffernde Komponente umfasst.

4. Zusammensetzung nach Anspruch 1, wobei die Polysucrose und die Trehalose in der Zusammensetzung in einem Verhältnis von 1:1 bis 5:1 vorhanden sind.

5. Zusammensetzung nach Anspruch 3, wobei die puffernde Komponente ausgewählt ist aus der Gruppe, bestehend aus phosphatgepufferter Kochsalzlösung (PBS), Bicarbonat/Kohlensäure, wie einem Natriumbicarbonatpuffer, N-2-Hydroxyethylpiperazin-N'-2-ethansulfonsäure (HEPES), ein Puffer auf Tris-Basis, eine tris-gepufferte Kochsalzlösung (TBS) und Kombinationen davon, wobei die Zusammensetzung ferner ein Salz umfasst, das aus der Gruppe ausgewählt ist, bestehend aus einem Phosphatsalz, einem Natriumsalz, einem Kaliumsalz, einem Calciumsalz, einem Magnesiumsalz und Kombinationen davon; und wobei die Zusammensetzung eine hämostatische Zusammensetzung ist.

6. Zusammensetzung nach Anspruch 1, wobei das DMSO in einer Menge von 0,5 % Vol./Vol. bis 2 % Vol. in der Zusammensetzung vorhanden ist.

7. Verfahren zur Herstellung einer kryokonservierten Thrombozytenzusammensetzung, umfassend:
Erhalten von Thrombozyten, die in einem flüssigen Medium enthalten sind;
Suspendieren der Thrombozyten in einem Suspensionsmedium, das ein Kälteschutzmittel umfasst, das DMSO in einer Menge von weniger als 6 % Vol./Vol. in der Zusammensetzung umfasst, wobei das Kälteschutzmittel ferner eine Kombination aus einem Polysaccharid und einem Disaccharid umfasst, wobei das Polysaccharid ein Polysaccharidpolymer ist, das zu 3 % bis 10 % Vol. vorliegt, und wobei das Disaccharid Trehalose ist, die zu 1,5 % bis 5 % Gew./Vol. vorliegt, um die Thrombozyten mit dem Kälteschutzmittel zu beladen, um eine mit Kälteschutzmittel beladene Thrombozytensuspension zu bilden; und
Aussetzen der mit einem Kryoprotektivum beladenen Thrombozytensuspension einer Temperatur von weniger als -1 °C für eine Zeit, die ausreicht, um die mit einem Kryoprotektivum beladene Thrombozytensuspension zu gefrieren, wodurch die kryokonservierte Thrombozytenzusammensetzung gebildet wird, wobei das Verfahren keinen Lyophilisierungsschritt umfasst.

8. Verfahren nach Anspruch 7, wobei das DMSO in einer Menge von weniger als 1 % Vol./Vol. oder von 1 % Vol./Vol. bis 3 % Vol./Vol. vorliegt.

9. Verfahren nach Anspruch 7, wobei das Verfahren ferner die Lagerung der Thrombozyten für mindestens 6 Monate bei einer Temperatur von -80 °C +/- 10 °C umfasst, wobei nach der Lagerung die kryokonservierte Thrombozytenzusammensetzung eine angemessene hämostatische Funktion und eine primäre koagulopathische Funktion bereitstellt.

10. Verfahren nach Anspruch 7, wobei das Suspensionsmedium ferner ein organisches Lösungsmittel umfasst, wobei das organische Lösungsmittel in einer Menge von 0,8 % bis 8 % Vol./Vol. des Suspensionsmediums vorhanden ist, wobei das organische Lösungsmittel einen kurzkettigen Alkohol umfasst, der aus der Gruppe ausgewählt ist, die aus Methanol, Ethanol, Propanol, 1-Propanol, 2-Propanol und Kombinationen davon besteht.

11. Verfahren nach einem der Ansprüche 7 bis 10, wobei das Suspensionsmedium einen pH-Wert von 4,0 bis 7,0 aufweist.

12. Verfahren nach einem der Ansprüche 7 bis 10, wobei das Suspensionsmedium einen pH-Wert von 6,2 bis 8,0 aufweist und wobei das Suspendieren der Thrombozyten in dem Suspensionsmedium bei einer Temperatur von 20 °C bis 42 °C durchgeführt wird.

13. Zusammensetzung nach einem der Ansprüche 1 bis 6, wobei die kryokonservierten Thrombozyten Thrombozyten umfassen, die von einem Menschen stammen.

14. Zusammensetzung nach einem der Ansprüche 1 bis 6 oder 13, wobei mindestens 50 % der kryokonservierten Thrombozyten im Größenbereich von 0,3 µm bis 5,0 µm liegen.

15. Zusammensetzung nach einem der Ansprüche 1 bis 6 oder 13, wobei das Kälteschutzmittel ferner Glycerin in einer Menge von 0,1 % Vol./Vol. bis 10 % Vol./Vol. in der Zusammensetzung umfasst.

## Revendications

1. Composition comprenant :
des plaquettes cryoconservées, dans laquelle les plaquettes cryoconservées sont une suspension de plaquettes congelées ; et
un cryoprotecteur comprenant du diméthylsulfoxyde (DMSO) en une quantité inférieure à 6 % v/v dans la composition, dans laquelle le cryoprotecteur comprenant en outre une combinaison d'un polysaccharide et d'un disaccharide, dans laquelle le polysaccharide est un polymère de polysucrose ayant une concentration dans la plage de 3 % à 10 % p/v dans la composition, et dans laquelle le disaccharide est le tréhalose en une quantité dans la plage de 1,5 % à 5 % p/v dans la composition, et dans laquelle la composition n'est pas obtenue à l'aide d'une étape de lyophilisation.

2. Composition selon la revendication 1, dans laquelle le DMSO est présent en une quantité de 0,5 % v/v à 1 % v/v ou de 1 % v/v à 3 % v/v.

3. Composition selon la revendication 1, dans laquelle la composition comprend en outre un composant tampon.

4. Composition selon la revendication 1, dans laquelle le polysucrose et le tréhalose sont présents dans la composition dans un rapport de 1:1 à 5:1.

5. Composition selon la revendication 3, dans laquelle le composant tampon est choisi dans le groupe constitué par un tampon salin au phosphate (PBS), un bicarbonate/acide carbonique, tel qu'un tampon au bicarbonate de sodium, l'acide N-2-hydroxyéthylpipérazine-N'-2-éthanesulfonique (HEPES), un tampon à base de tris, un tampon salin au tris (TBS), et des combinaisons de ceux-ci, dans laquelle la composition comprend en outre un sel choisi dans le groupe constitué par un sel de phosphate, un sel de sodium, un sel de potassium, un sel de calcium, un sel de magnésium, et des combinaisons de ceux-ci ; et dans laquelle la composition est une composition hémostatique.

6. Composition selon la revendication 1, dans laquelle le DMSO est présent en une quantité de 0,5 % v/v à 2 % v/v dans la composition.

7. Procédé de fabrication d'une composition de plaquettes cryoconservées, comprenant :
l'obtention de plaquettes contenues dans un milieu liquide ;
la mise en suspension des plaquettes dans un milieu de suspension comprenant un cryoprotecteur comprenant du DMSO en une quantité inférieure à 6 % v/v dans la composition, dans lequel le cryoprotecteur comprend en outre une combinaison d'un polysaccharide et d'un disaccharide, dans lequel le polysaccharide est un polymère de polysucrose présent à raison de 3 % à 10 % p/v, et dans lequel le disaccharide est du tréhalose présent à raison de 1,5 % à 5 % p/v pour charger les plaquettes avec le cryoprotecteur afin de former une suspension de plaquettes chargées de cryoprotecteur ; et
la soumission de la suspension de plaquettes chargées de cryoprotecteur à une température inférieure à -1 °C, pendant une durée suffisante pour congeler la suspension de plaquettes chargées de cryoprotecteur, formant ainsi la composition de plaquettes cryoconservées, dans lequel le procédé ne comprenant pas d'étape de lyophilisation.

8. Procédé selon la revendication 7, dans lequel le DMSO est présent en une quantité inférieure à 1 % v/v ou de 1 % v/v à 3 % v/v.

9. Procédé selon la revendication 7, dans lequel le procédé comprend en outre le stockage des plaquettes pendant au moins 6 mois à une température de -80 °C +/-10 °C, dans lequel, après le stockage, la composition de plaquettes cryoconservées fournit une fonction hémostatique adéquate et une fonction coagulopathique primaire.

10. Procédé selon la revendication 7, dans lequel le milieu de suspension comprend en outre un solvant organique, dans lequel le solvant organique est présent en une quantité de 0,8 % à 8 % v/v du milieu de suspension, dans lequel le solvant organique comprend un alcool à chaîne courte choisi dans le groupe constitué par le méthanol, l'éthanol, le propanol, le 1-propanol, le 2-propanol et des combinaisons de ceux-ci.

11. Procédé selon l'une quelconque des revendications 7 à 10, dans lequel le milieu de suspension a un pH de 4,0 à 7,0.

12. Procédé selon l'une quelconque des revendications 7 à 10, dans lequel le milieu de suspension a un pH de 6,2 à 8,0, et dans lequel la mise en suspension des plaquettes dans le milieu de suspension est effectuée à une température de 20 °C à 42 °C.

13. Composition selon l'une quelconque des revendications 1 à 6, dans laquelle les plaquettes cryoconservées comprennent des plaquettes dérivées d'un être humain.

14. Composition selon l'une quelconque des revendications 1 à 6, ou 13, dans laquelle au moins 50 % des plaquettes cryoconservées sont dans la plage de tailles de 0,3 µm à 5,0 µm.

15. Composition selon l'une quelconque des revendications 1 à 6, ou 13, dans laquelle le cryoprotecteur comprend en outre du glycérol en une quantité de 0,1 % v/v à 10 % v/v dans la composition.
